# EUROPÄISCHE PATENTANMELDUNG

(11) **EP 1 170 364 A1**
(43) Veröffentlichungstag der Anmeldung: **09.01.2002**
(21) Anmeldenummer: 00114622.4
(22) Anmeldetag: 07.07.2000
(51) Int. Cl.: C12N 15/12, C12N 15/52, C12N 15/62, C12N 15/63, C12N 15/85, C12N 9/00, C12N 5/10, C07K 14/72

(54) **Mutationen kryptischer Spleissstellen in Cre und Cre-Fusionsproteinen zur Verbesserung der Expression und Induzierbarkeit**

(71) Anmelder: ARTEMIS Pharmaceuticals GmbH, 51063 Köln (DE)
(72) Erfinder: Edenhofer, Frank Oliver Stefan, 50374 Köln (DE); Wunderlich, Thomas, 50668 Köln (DE); Rajewsky, Klaus, 50931 Köln (DE)
(74) Vertreter: Helbing, Jörg, Dr. Dipl.-Chem.

(57) **Zusammenfassung**

Die vorliegende Erfindung betrifft eine DNA-Sequenz, die für eine Mutante der Bakteriophagen P1-Rekombinase kodiert, in der eine spezielle kryptische Spleißstelle durch eine die Cre-Proteinsequenz ändernde Basenmutation ausgeschaltet ist, sowie eine DNA-Sequenz, die für ein Fusionsprotein aus der genannten Cre-Mutanten und einer ligandenbindenden Domäne eines Rezeptorproteins kodiert. Die Erfindung betrifft weiterhin Vektoren, Mikroorganismen und transgene Lebewesen, die solch eine DNA-Sequenz enthalten, sowie Cre-Mutanten oder Cre-Fusionsproteine, die von diesen DNA-Sequenzen kodiert werden.

SEQUENZPROTOKOLL

<110> ARTEMIS Pharmaceuticals GmbH

<120> Mutation kryptischer Spleißstellen in Cre und
      Cre-Fusionsproteinen zur Verbesserung der Expression
      und Induzierbarkeit

<130> 992876de/JH/ml

<140>
<141>

<160> 23

<170> PatentIn Ver. 2.1


<210> 1
<211> 1032
<212> DNA
<213> Bacteriophage P1

<220>
<221> CDS
<222> (1)..(1029)

<400> 1
atg tcc aat tta ctg acc gta cac caa aat ttg cct gca tta ccg gtc    48
Met Ser Asn Leu Leu Thr Val His Gln Asn Leu Pro Ala Leu Pro Val
  1               5                  10                  15

gat gca acg agt gat gag gtt cgc aag aac ctg atg gac atg ttc agg    96
Asp Ala Thr Ser Asp Glu Val Arg Lys Asn Leu Met Asp Met Phe Arg
             20                  25                  30

gat cgc cag gcg ttt tct gag cat acc tgg aaa atg ctt ctg tcc gtt    144
Asp Arg Gln Ala Phe Ser Glu His Thr Trp Lys Met Leu Leu Ser Val
         35                  40                  45

tgc cgg tcg tgg gcg gca tgg tgc aag ttg aat aac cgg aaa tgg ttt    192
Cys Arg Ser Trp Ala Ala Trp Cys Lys Leu Asn Asn Arg Lys Trp Phe
     50                  55                  60

ccc gca gaa cct gaa gat gtt cgc gat tat ctt cta tat ctt cag gcg    240
Pro Ala Glu Pro Glu Asp Val Arg Asp Tyr Leu Leu Tyr Leu Gln Ala
 65                  70                  75                  80

cgc ggt ctg gca gta aaa act atc cag caa cat ttg ggc cag cta aac    288
Arg Gly Leu Ala Val Lys Thr Ile Gln Gln His Leu Gly Gln Leu Asn
                 85                  90                  95

atg ctt cat cgt cgg tcc ggg ctg cca cga cca agt gac agc aat gct    336
Met Leu His Arg Arg Ser Gly Leu Pro Arg Pro Ser Asp Ser Asn Ala
            100                 105                 110

gtt tca ctg gtt atg cgg cgg atc cga aaa gaa aac gtt gat gcc ggt    384
Val Ser Leu Val Met Arg Arg Ile Arg Lys Glu Asn Val Asp Ala Gly
        115                 120                 125

gaa cgt gca aaa cag gct cta gcg ttc gaa cgc act gat ttc gac cag     432
Glu Arg Ala Lys Gln Ala Leu Ala Phe Glu Arg Thr Asp Phe Asp Gln
    130                 135                 140

gtt cgt tca ctc atg gaa aat agc gat cgc tgc cag gat ata cgt aat     480
Val Arg Ser Leu Met Glu Asn Ser Asp Arg Cys Gln Asp Ile Arg Asn
145                 150                 155                 160

ctg gca ttt ctg ggg att gct tat aac acc ctg tta cgt ata gcc gaa     528
Leu Ala Phe Leu Gly Ile Ala Tyr Asn Thr Leu Leu Arg Ile Ala Glu
                165                 170                 175

att gcc agg atc agg gtt aaa gat atc tca cgt act gac ggt ggg aga     576
Ile Ala Arg Ile Arg Val Lys Asp Ile Ser Arg Thr Asp Gly Gly Arg
            180                 185                 190

atg tta atc cat att ggc aga acg aaa acg ctg gtt agc acc gca ggt     624
Met Leu Ile His Ile Gly Arg Thr Lys Thr Leu Val Ser Thr Ala Gly
        195                 200                 205

gta gag aag gca ctt agc ctg ggg gta act aaa ctg gtc gag cga tgg     672
Val Glu Lys Ala Leu Ser Leu Gly Val Thr Lys Leu Val Glu Arg Trp
    210                 215                 220

att tcc gtc tct ggt gta gct gat gat ccg aat aac tac ctg ttt tgc     720
Ile Ser Val Ser Gly Val Ala Asp Asp Pro Asn Asn Tyr Leu Phe Cys
225                 230                 235                 240

cgg gtc aga aaa aat ggt gtt gcc gcg cca tct gcc acc agc cag cta     768
Arg Val Arg Lys Asn Gly Val Ala Ala Pro Ser Ala Thr Ser Gln Leu
                245                 250                 255

tca act cgc gcc ctg gaa ggg att ttt gaa gca act cat cga ttg att     816
Ser Thr Arg Ala Leu Glu Gly Ile Phe Glu Ala Thr His Arg Leu Ile
            260                 265                 270

tac ggc gct aag gat gac tct ggt cag aga tac ctg gcc tgg tct gga     864
Tyr Gly Ala Lys Asp Asp Ser Gly Gln Arg Tyr Leu Ala Trp Ser Gly
        275                 280                 285

cac agt gcc cgt gtc gga gcc gcg cga gat atg gcc cgc gct gga gtt     912
His Ser Ala Arg Val Gly Ala Ala Arg Asp Met Ala Arg Ala Gly Val
    290                 295                 300

tca ata ccg gag atc atg caa gct ggt ggc tgg acc aat gta aat att     960
Ser Ile Pro Glu Ile Met Gln Ala Gly Gly Trp Thr Asn Val Asn Ile
305                 310                 315                 320

gtc atg aac tat atc cgt aac ctg gat agt gaa aca ggg gca atg gtg     1008
Val Met Asn Tyr Ile Arg Asn Leu Asp Ser Glu Thr Gly Ala Met Val
                325                 330                 335

cgc ctg ctg gaa gat ggc gat tag                                     1032
Arg Leu Leu Glu Asp Gly Asp
            340

<210> 2
<211> 343
<212> PRT
<213> Bacteriophage P1

<400> 2
Met Ser Asn Leu Leu Thr Val His Gln Asn Leu Pro Ala Leu Pro Val
1               5                   10                  15

Asp Ala Thr Ser Asp Glu Val Arg Lys Asn Leu Met Asp Met Phe Arg
            20                  25                  30

Asp Arg Gln Ala Phe Ser Glu His Thr Trp Lys Met Leu Leu Ser Val
        35                  40                  45

Cys Arg Ser Trp Ala Ala Trp Cys Lys Leu Asn Asn Arg Lys Trp Phe
    50                  55                  60

Pro Ala Glu Pro Glu Asp Val Arg Asp Tyr Leu Leu Tyr Leu Gln Ala
65                  70                  75                  80

Arg Gly Leu Ala Val Lys Thr Ile Gln Gln His Leu Gly Gln Leu Asn
                85                  90                  95

Met Leu His Arg Arg Ser Gly Leu Pro Arg Pro Ser Asp Ser Asn Ala
            100                 105                 110

Val Ser Leu Val Met Arg Arg Ile Arg Lys Glu Asn Val Asp Ala Gly
        115                 120                 125

Glu Arg Ala Lys Gln Ala Leu Ala Phe Glu Arg Thr Asp Phe Asp Gln
    130                 135                 140

Val Arg Ser Leu Met Glu Asn Ser Asp Arg Cys Gln Asp Ile Arg Asn
145                 150                 155                 160

Leu Ala Phe Leu Gly Ile Ala Tyr Asn Thr Leu Leu Arg Ile Ala Glu
                165                 170                 175

Ile Ala Arg Ile Arg Val Lys Asp Ile Ser Arg Thr Asp Gly Gly Arg
            180                 185                 190

Met Leu Ile His Ile Gly Arg Thr Lys Thr Leu Val Ser Thr Ala Gly
        195                 200                 205

Val Glu Lys Ala Leu Ser Leu Gly Val Thr Lys Leu Val Glu Arg Trp
    210                 215                 220

Ile Ser Val Ser Gly Val Ala Asp Asp Pro Asn Asn Tyr Leu Phe Cys
225                 230                 235                 240

Arg Val Arg Lys Asn Gly Val Ala Ala Pro Ser Ala Thr Ser Gln Leu
                245                 250                 255

Ser Thr Arg Ala Leu Glu Gly Ile Phe Glu Ala Thr His Arg Leu Ile
            260                 265                 270

Tyr Gly Ala Lys Asp Asp Ser Gly Gln Arg Tyr Leu Ala Trp Ser Gly
        275                 280                 285

His Ser Ala Arg Val Gly Ala Ala Arg Asp Met Ala Arg Ala Gly Val
    290                 295                 300

Ser Ile Pro Glu Ile Met Gln Ala Gly Gly Trp Thr Asn Val Asn Ile
305                 310                 315                 320

Val Met Asn Tyr Ile Arg Asn Leu Asp Ser Glu Thr Gly Ala Met Val
                325                 330                 335

Arg Leu Leu Glu Asp Gly Asp
            340

<210> 3
<211> 2802
<212> DNA
<213> Homo sapiens

<220>
<221> CDS
<222> (1)..(2799)

<400> 3

atg act gag ctg aag gca aag ggt ccc cgg gct ccc cac gtg gcg ggc      48
Met Thr Glu Leu Lys Ala Lys Gly Pro Arg Ala Pro His Val Ala Gly
1               5                   10                  15

ggc ccg ccc tcc ccc gag gtc gga tcc cca ctg ctg tgt cgc cca gcc      96
Gly Pro Pro Ser Pro Glu Val Gly Ser Pro Leu Leu Cys Arg Pro Ala
            20                  25                  30

gca ggt ccg ttc ccg ggg agc cag acc tcg gac acc ttg cct gaa gtt     144
Ala Gly Pro Phe Pro Gly Ser Gln Thr Ser Asp Thr Leu Pro Glu Val
        35                  40                  45

tcg gcc ata cct atc tcc ctg gac ggg cta ctc ttc cct cgg ccc tgc     192
Ser Ala Ile Pro Ile Ser Leu Asp Gly Leu Leu Phe Pro Arg Pro Cys
    50                  55                  60

cag gga cag gac ccc tcc gac gaa aag acg cag gac cag cag tcg ctg     240
Gln Gly Gln Asp Pro Ser Asp Glu Lys Thr Gln Asp Gln Gln Ser Leu
65                  70                  75                  80

tcg gac gtg gag ggc gca tat tcc aga gct gaa gct aca agg ggt gct     288
Ser Asp Val Glu Gly Ala Tyr Ser Arg Ala Glu Ala Thr Arg Gly Ala
                85                  90                  95

gga ggc agc agt tct agt ccc cca gaa aag gac agc gga ctg ctg gac     336
Gly Gly Ser Ser Ser Ser Pro Pro Glu Lys Asp Ser Gly Leu Leu Asp
            100                 105                 110

agt gtc ttg gac act ctg ttg gcg ccc tca ggt ccc ggg cag agc caa     384
Ser Val Leu Asp Thr Leu Leu Ala Pro Ser Gly Pro Gly Gln Ser Gln
        115                 120                 125

ccc agc cct ccc gcc tgc gag gtc acc agc tct tgg tgc ctg ttt ggc     432
Pro Ser Pro Pro Ala Cys Glu Val Thr Ser Ser Trp Cys Leu Phe Gly
    130                 135                 140

ccc gaa ctt ccc gaa gat cca ccg gct gcc ccc gcc acc cag cgg gtg     480
Pro Glu Leu Pro Glu Asp Pro Pro Ala Ala Pro Ala Thr Gln Arg Val
145                 150                 155                 160

ttg tcc ccg ctc atg agc cgg tcc ggg tgc aag gtt gga gac agc tcc 528
Leu Ser Pro Leu Met Ser Arg Ser Gly Cys Lys Val Gly Asp Ser Ser
                165                 170                 175

ggg acg gca gct gcc cat aaa gtg ctg ccc cgg ggc ctg tca cca gcc 576
Gly Thr Ala Ala Ala His Lys Val Leu Pro Arg Gly Leu Ser Pro Ala
            180                 185                 190

cgg cag ctg ctg ctc ccg gcc tct gag agc cct cac tgg tcc ggg gcc 624
Arg Gln Leu Leu Leu Pro Ala Ser Glu Ser Pro His Trp Ser Gly Ala
        195                 200                 205

cca gtg aag ccg tct ccg cag gcc gct gcg gtg gag gtt gag gag gag 672
Pro Val Lys Pro Ser Pro Gln Ala Ala Ala Val Glu Val Glu Glu Glu
    210                 215                 220

gat ggc tct gag tcc gag gag tct gcg ggt ccg ctt ctg aag ggc aaa 720
Asp Gly Ser Glu Ser Glu Glu Ser Ala Gly Pro Leu Leu Lys Gly Lys
225                 230                 235                 240

cct cgg gct ctg ggt ggc gcg gcg gct gga gga gga gcc gcg gct gtc 768
Pro Arg Ala Leu Gly Gly Ala Ala Ala Gly Gly Gly Ala Ala Ala Val
                245                 250                 255

ccg ccg ggg gcg gca gca gga ggc gtc gcc ctg gtc ccc aag gaa gat 816
Pro Pro Gly Ala Ala Ala Gly Gly Val Ala Leu Val Pro Lys Glu Asp
            260                 265                 270

tcc cgc ttc tca gcg ccc agg gtc gcc ctg gtg gag cag gac gcg ccg 864
Ser Arg Phe Ser Ala Pro Arg Val Ala Leu Val Glu Gln Asp Ala Pro
        275                 280                 285

atg gcg ccc ggg cgc tcc ccg ctg gcc acc acg gtg atg gat ttc atc 912
Met Ala Pro Gly Arg Ser Pro Leu Ala Thr Thr Val Met Asp Phe Ile
    290                 295                 300

cac gtg cct atc ctg cct ctc aat cac gcc tta ttg gca gcc cgc act 960
His Val Pro Ile Leu Pro Leu Asn His Ala Leu Leu Ala Ala Arg Thr
305                 310                 315                 320

cgg cag ctg ctg gaa gac gaa agt tac gac ggc ggg gcc ggg gct gcc 1008
Arg Gln Leu Leu Glu Asp Glu Ser Tyr Asp Gly Gly Ala Gly Ala Ala
                325                 330                 335

agc gcc ttt gcc ccg ccg cgg agt tca ccc tgt gcc tcg tcc acc ccg 1056
Ser Ala Phe Ala Pro Pro Arg Ser Ser Pro Cys Ala Ser Ser Thr Pro
            340                 345                 350

gtc gct gta ggc gac ttc ccc gac tgc gcg tac ccg ccc gac gcc gag 1104
Val Ala Val Gly Asp Phe Pro Asp Cys Ala Tyr Pro Pro Asp Ala Glu
        355                 360                 365

ccc aag gac gac gcg tac cct ctc tat agc gac ttc cag ccg ccc gct 1152
Pro Lys Asp Asp Ala Tyr Pro Leu Tyr Ser Asp Phe Gln Pro Pro Ala
    370                 375                 380

cta aag ata aag gag gag gag gaa ggc gcg gag gcc tcc gcg cgc tcc 1200
Leu Lys Ile Lys Glu Glu Glu Glu Gly Ala Glu Ala Ser Ala Arg Ser
385                 390                 395                 400

ccg cgt tcc tac ctt gtg gcc ggt gcc aac ccc gca gcc ttc ccg gat     1248
Pro Arg Ser Tyr Leu Val Ala Gly Ala Asn Pro Ala Ala Phe Pro Asp
                405                 410                 415

ttc ccg ttg ggg cca ccg ccc ccg ctg ccg ccg cga gcg acc cca tcc     1296
Phe Pro Leu Gly Pro Pro Pro Pro Leu Pro Pro Arg Ala Thr Pro Ser
            420                 425                 430

aga ccc ggg gaa gcg gcg gtg acg gcc gca ccc gcc agt gcc tca gtc     1344
Arg Pro Gly Glu Ala Ala Val Thr Ala Ala Pro Ala Ser Ala Ser Val
        435                 440                 445

tcg tct gcg tcc tcc tcg ggg tcg acc ctg gag tgc atc ctg tac aaa     1392
Ser Ser Ala Ser Ser Ser Gly Ser Thr Leu Glu Cys Ile Leu Tyr Lys
    450                 455                 460

gcg gag ggc gcg ccg ccc cag cag ggc ccg ttc gcg ccg ccg ccc tgc     1440
Ala Glu Gly Ala Pro Pro Gln Gln Gly Pro Phe Ala Pro Pro Pro Cys
465                 470                 475                 480

aag gcg ccg ggc gcg agc ggc tgc ctg ctc ccg cgg gac ggc ctg ccc     1488
Lys Ala Pro Gly Ala Ser Gly Cys Leu Leu Pro Arg Asp Gly Leu Pro
                485                 490                 495

tcc acc tcc gcc tct gcc gcc gcc gcc ggg gcg gcc ccc gcg ctc tac     1536
Ser Thr Ser Ala Ser Ala Ala Ala Ala Gly Ala Ala Pro Ala Leu Tyr
            500                 505                 510

cct gca ctc ggc ctc aac ggg ctc ccg cag ctc ggc tac cag gcc gcc     1584
Pro Ala Leu Gly Leu Asn Gly Leu Pro Gln Leu Gly Tyr Gln Ala Ala
        515                 520                 525

gtg ctc aag gag ggc ctg ccg cag gtc tac ccg ccc tat ctc aac tac     1632
Val Leu Lys Glu Gly Leu Pro Gln Val Tyr Pro Pro Tyr Leu Asn Tyr
    530                 535                 540

ctg agg ccg gat tca gaa gcc agc cag agc cca caa tac agc ttc gag     1680
Leu Arg Pro Asp Ser Glu Ala Ser Gln Ser Pro Gln Tyr Ser Phe Glu
545                 550                 555                 560

tca tta cct cag aag att tgt tta atc tgt ggg gat gaa gca tca ggc     1728
Ser Leu Pro Gln Lys Ile Cys Leu Ile Cys Gly Asp Glu Ala Ser Gly
                565                 570                 575

tgt cat tat ggt gtc ctt acc tgt ggg agc tgt aag gtc ttc ttt aag     1776
Cys His Tyr Gly Val Leu Thr Cys Gly Ser Cys Lys Val Phe Phe Lys
            580                 585                 590

agg gca atg gaa ggg cag cac aac tac tta tgt gct gga aga aat gac     1824
Arg Ala Met Glu Gly Gln His Asn Tyr Leu Cys Ala Gly Arg Asn Asp
        595                 600                 605

tgc atc gtt gat aaa atc cgc aga aaa aac tgc cca gca tgt cgc ctt     1872
Cys Ile Val Asp Lys Ile Arg Arg Lys Asn Cys Pro Ala Cys Arg Leu
    610                 615                 620

aga aag tgc tgt cag gct ggc atg gtc ctt gga ggt cga aaa ttt aaa     1920
Arg Lys Cys Cys Gln Ala Gly Met Val Leu Gly Gly Arg Lys Phe Lys
625                 630                 635                 640

aag ttc aat aaa gtc aga gtt gtg aga gca ctg gat gct gtt gct ctc     1968
Lys Phe Asn Lys Val Arg Val Val Arg Ala Leu Asp Ala Val Ala Leu
                645                 650                 655

cca cag cca gtg ggc gtt cca aat gaa agc caa gcc cta agc cag aga     2016
Pro Gln Pro Val Gly Val Pro Asn Glu Ser Gln Ala Leu Ser Gln Arg
            660                 665                 670

ttc act ttt tca cca ggt caa gac ata cag ttg att cca cca ctg atc     2064
Phe Thr Phe Ser Pro Gly Gln Asp Ile Gln Leu Ile Pro Pro Leu Ile
        675                 680                 685

aac ctg tta atg agc att gaa cca gat gtg atc tat gca gga cat gac     2112
Asn Leu Leu Met Ser Ile Glu Pro Asp Val Ile Tyr Ala Gly His Asp
    690                 695                 700

aac aca aaa cct gac acc tcc agt tct ttg ctg aca agt ctt aat caa     2160
Asn Thr Lys Pro Asp Thr Ser Ser Ser Leu Leu Thr Ser Leu Asn Gln
705                 710                 715                 720

cta ggc gag agg caa ctt ctt tca gta gtc aag tgg tct aaa tca ttg     2208
Leu Gly Glu Arg Gln Leu Leu Ser Val Val Lys Trp Ser Lys Ser Leu
                725                 730                 735

cca ggt ttt cga aac tta cat att gat gac cag ata act ctc att cag     2256
Pro Gly Phe Arg Asn Leu His Ile Asp Asp Gln Ile Thr Leu Ile Gln
            740                 745                 750

tat tct tgg atg agc tta atg gtg ttt ggt cta gga tgg aga tcc tac     2304
Tyr Ser Trp Met Ser Leu Met Val Phe Gly Leu Gly Trp Arg Ser Tyr
        755                 760                 765

aaa cac gtc agt ggg cag atg ctg tat ttt gca cct gat cta ata cta     2352
Lys His Val Ser Gly Gln Met Leu Tyr Phe Ala Pro Asp Leu Ile Leu
    770                 775                 780

aat gaa cag cgg atg aaa gaa tca tca ttc tat tca tta tgc ctt acc     2400
Asn Glu Gln Arg Met Lys Glu Ser Ser Phe Tyr Ser Leu Cys Leu Thr
785                 790                 795                 800

atg tgg cag atc cca cag gag ttt gtc aag ctt caa gtt agc caa gaa     2448
Met Trp Gln Ile Pro Gln Glu Phe Val Lys Leu Gln Val Ser Gln Glu
                805                 810                 815

gag ttc ctc tgt atg aaa gta ttg tta ctt ctt aat aca att cct ttg     2496
Glu Phe Leu Cys Met Lys Val Leu Leu Leu Leu Asn Thr Ile Pro Leu
            820                 825                 830

gaa ggg cta cga agt caa acc cag ttt gag gag atg agg tca agc tac     2544
Glu Gly Leu Arg Ser Gln Thr Gln Phe Glu Glu Met Arg Ser Ser Tyr
        835                 840                 845

att aga gag ctc atc aag gca att ggt ttg agg caa aaa gga gtt gtg     2592
Ile Arg Glu Leu Ile Lys Ala Ile Gly Leu Arg Gln Lys Gly Val Val
    850                 855                 860

tcg agc tca cag cgt ttc tat caa ctt aca aaa ctt ctt gat aac ttg     2640
Ser Ser Ser Gln Arg Phe Tyr Gln Leu Thr Lys Leu Leu Asp Asn Leu
865                 870                 875                 880

cat gat ctt gtc aaa caa ctt cat ctg tac tgc ttg aat aca ttt atc     2688
His Asp Leu Val Lys Gln Leu His Leu Tyr Cys Leu Asn Thr Phe Ile
                885                 890                 895

cag tcc cgg gca ctg agt gtt gaa ttt cca gaa atg atg tct gaa gtt     2736
Gln Ser Arg Ala Leu Ser Val Glu Phe Pro Glu Met Met Ser Glu Val
            900                 905                 910

att gct gca caa tta ccc aag ata ttg gca ggg atg gtg aaa ccc ctt     2784
Ile Ala Ala Gln Leu Pro Lys Ile Leu Ala Gly Met Val Lys Pro Leu
        915                 920                 925

ctc ttt cat aaa aag tga                                             2802
Leu Phe His Lys Lys
    930


<210> 4
<211> 933
<212> PRT
<213> Homo sapiens

<400> 4
Met Thr Glu Leu Lys Ala Lys Gly Pro Arg Ala Pro His Val Ala Gly
1               5                   10                  15

Gly Pro Pro Ser Pro Glu Val Gly Ser Pro Leu Leu Cys Arg Pro Ala
            20                  25                  30

Ala Gly Pro Phe Pro Gly Ser Gln Thr Ser Asp Thr Leu Pro Glu Val
        35                  40                  45

Ser Ala Ile Pro Ile Ser Leu Asp Gly Leu Leu Phe Pro Arg Pro Cys
    50                  55                  60

Gln Gly Gln Asp Pro Ser Asp Glu Lys Thr Gln Asp Gln Gln Ser Leu
65                  70                  75                  80

Ser Asp Val Glu Gly Ala Tyr Ser Arg Ala Glu Ala Thr Arg Gly Ala
                85                  90                  95

Gly Gly Ser Ser Ser Ser Pro Pro Glu Lys Asp Ser Gly Leu Leu Asp
            100                 105                 110

Ser Val Leu Asp Thr Leu Leu Ala Pro Ser Gly Pro Gly Gln Ser Gln
        115                 120                 125

Pro Ser Pro Pro Ala Cys Glu Val Thr Ser Ser Trp Cys Leu Phe Gly
    130                 135                 140

Pro Glu Leu Pro Glu Asp Pro Pro Ala Ala Pro Ala Thr Gln Arg Val
145                 150                 155                 160

Leu Ser Pro Leu Met Ser Arg Ser Gly Cys Lys Val Gly Asp Ser Ser
                165                 170                 175

Gly Thr Ala Ala Ala His Lys Val Leu Pro Arg Gly Leu Ser Pro Ala
            180                 185                 190

Arg Gln Leu Leu Leu Pro Ala Ser Glu Ser Pro His Trp Ser Gly Ala
        195                 200                 205

Pro Val Lys Pro Ser Pro Gln Ala Ala Ala Val Glu Val Glu Glu Glu
    210                 215                 220

Asp Gly Ser Glu Ser Glu Glu Ser Ala Gly Pro Leu Leu Lys Gly Lys
225                 230                 235                 240

Pro Arg Ala Leu Gly Gly Ala Ala Ala Gly Gly Gly Ala Ala Ala Val
                245                 250                 255

Pro Pro Gly Ala Ala Ala Gly Gly Val Ala Leu Val Pro Lys Glu Asp
            260                 265                 270

Ser Arg Phe Ser Ala Pro Arg Val Ala Leu Val Glu Gln Asp Ala Pro
        275                 280                 285

Met Ala Pro Gly Arg Ser Pro Leu Ala Thr Thr Val Met Asp Phe Ile
    290                 295                 300

His Val Pro Ile Leu Pro Leu Asn His Ala Leu Leu Ala Ala Arg Thr
305                 310                 315                 320

Arg Gln Leu Leu Glu Asp Glu Ser Tyr Asp Gly Gly Ala Gly Ala Ala
                325                 330                 335

Ser Ala Phe Ala Pro Pro Arg Ser Ser Pro Cys Ala Ser Ser Thr Pro
            340                 345                 350

Val Ala Val Gly Asp Phe Pro Asp Cys Ala Tyr Pro Pro Asp Ala Glu
        355                 360                 365

Pro Lys Asp Asp Ala Tyr Pro Leu Tyr Ser Asp Phe Gln Pro Pro Ala
    370                 375                 380

Leu Lys Ile Lys Glu Glu Glu Glu Gly Ala Glu Ala Ser Ala Arg Ser
385                 390                 395                 400

Pro Arg Ser Tyr Leu Val Ala Gly Ala Asn Pro Ala Ala Phe Pro Asp
                405                 410                 415

Phe Pro Leu Gly Pro Pro Pro Pro Leu Pro Pro Arg Ala Thr Pro Ser
            420                 425                 430

Arg Pro Gly Glu Ala Ala Val Thr Ala Ala Pro Ala Ser Ala Ser Val
        435                 440                 445

Ser Ser Ala Ser Ser Ser Gly Ser Thr Leu Glu Cys Ile Leu Tyr Lys
    450                 455                 460

Ala Glu Gly Ala Pro Pro Gln Gln Gly Pro Phe Ala Pro Pro Pro Cys
465                 470                 475                 480

Lys Ala Pro Gly Ala Ser Gly Cys Leu Leu Pro Arg Asp Gly Leu Pro
                485                 490                 495

Ser Thr Ser Ala Ser Ala Ala Ala Ala Gly Ala Ala Pro Ala Leu Tyr
            500                 505                 510

Pro Ala Leu Gly Leu Asn Gly Leu Pro Gln Leu Gly Tyr Gln Ala Ala
        515                 520                 525

Val Leu Lys Glu Gly Leu Pro Gln Val Tyr Pro Pro Tyr Leu Asn Tyr
    530                 535                 540

Leu Arg Pro Asp Ser Glu Ala Ser Gln Ser Pro Gln Tyr Ser Phe Glu
545                 550                 555                 560

Ser Leu Pro Gln Lys Ile Cys Leu Ile Cys Gly Asp Glu Ala Ser Gly
                565                 570                 575

Cys His Tyr Gly Val Leu Thr Cys Gly Ser Cys Lys Val Phe Phe Lys
            580                 585                 590

Arg Ala Met Glu Gly Gln His Asn Tyr Leu Cys Ala Gly Arg Asn Asp
        595                 600                 605

Cys Ile Val Asp Lys Ile Arg Arg Lys Asn Cys Pro Ala Cys Arg Leu
    610                 615                 620

Arg Lys Cys Cys Gln Ala Gly Met Val Leu Gly Gly Arg Lys Phe Lys
625                 630                 635                 640

Lys Phe Asn Lys Val Arg Val Val Arg Ala Leu Asp Ala Val Ala Leu
                645                 650                 655

Pro Gln Pro Val Gly Val Pro Asn Glu Ser Gln Ala Leu Ser Gln Arg
            660                 665                 670

Phe Thr Phe Ser Pro Gly Gln Asp Ile Gln Leu Ile Pro Pro Leu Ile
        675                 680                 685

Asn Leu Leu Met Ser Ile Glu Pro Asp Val Ile Tyr Ala Gly His Asp
    690                 695                 700

Asn Thr Lys Pro Asp Thr Ser Ser Ser Leu Leu Thr Ser Leu Asn Gln
705                 710                 715                 720

Leu Gly Glu Arg Gln Leu Leu Ser Val Val Lys Trp Ser Lys Ser Leu
                725                 730                 735

Pro Gly Phe Arg Asn Leu His Ile Asp Asp Gln Ile Thr Leu Ile Gln
            740                 745                 750

Tyr Ser Trp Met Ser Leu Met Val Phe Gly Leu Gly Trp Arg Ser Tyr
        755                 760                 765

Lys His Val Ser Gly Gln Met Leu Tyr Phe Ala Pro Asp Leu Ile Leu
    770                 775                 780

Asn Glu Gln Arg Met Lys Glu Ser Ser Phe Tyr Ser Leu Cys Leu Thr
785                 790                 795                 800

Met Trp Gln Ile Pro Gln Glu Phe Val Lys Leu Gln Val Ser Gln Glu
                805                 810                 815

Glu Phe Leu Cys Met Lys Val Leu Leu Leu Leu Asn Thr Ile Pro Leu
            820                 825                 830

Glu Gly Leu Arg Ser Gln Thr Gln Phe Glu Glu Met Arg Ser Ser Tyr
        835                 840                 845

Ile Arg Glu Leu Ile Lys Ala Ile Gly Leu Arg Gln Lys Gly Val Val
    850                 855                 860

Ser Ser Ser Gln Arg Phe Tyr Gln Leu Thr Lys Leu Leu Asp Asn Leu
865                 870                 875                 880

His Asp Leu Val Lys Gln Leu His Leu Tyr Cys Leu Asn Thr Phe Ile
                885                 890                 895

Gln Ser Arg Ala Leu Ser Val Glu Phe Pro Glu Met Met Ser Glu Val
            900                 905                 910

Ile Ala Ala Gln Leu Pro Lys Ile Leu Ala Gly Met Val Lys Pro Leu
        915                 920                 925

Leu Phe His Lys Lys
    930


<210> 5
<211> 1725
<212> DNA
<213> Künstliche Sequenz

<220>
<223> Beschreibung der künstlichen Sequenz:
      Fusionsprotein Bacteriophage
      P1-Cre19V336A-PR676-914

<220>
<221> CDS
<222> (1)..(1722)

<400> 5
atg ggc gcc acg agt gat gag gtt cgc aag aac ctg atg gac atg ttc      48
Met Gly Ala Thr Ser Asp Glu Val Arg Lys Asn Leu Met Asp Met Phe
  1               5                  10                  15

agg gat cgc cag gcg ttt tct gag cat acc tgg aaa atg ctt ctg tcc      96
Arg Asp Arg Gln Ala Phe Ser Glu His Thr Trp Lys Met Leu Leu Ser
             20                  25                  30

gtt tgc cgg tcg tgg gcg gca tgg tgc aag ttg aat aac cgg aaa tgg     144
Val Cys Arg Ser Trp Ala Ala Trp Cys Lys Leu Asn Asn Arg Lys Trp
         35                  40                  45

ttt ccc gca gaa cct gaa gat gtt cgc gat tat ctt cta tat ctt cag     192
Phe Pro Ala Glu Pro Glu Asp Val Arg Asp Tyr Leu Leu Tyr Leu Gln
     50                  55                  60

gcg cgc ggt ctg gca gta aaa act atc cag caa cat ttg ggc cag cta     240
Ala Arg Gly Leu Ala Val Lys Thr Ile Gln Gln His Leu Gly Gln Leu
 65                  70                  75                  80

aac atg ctt cat cgt cgg tcc ggg ctg cca cga cca agt gac agc aat     288
Asn Met Leu His Arg Arg Ser Gly Leu Pro Arg Pro Ser Asp Ser Asn
                 85                  90                  95

gct gtt tca ctg gtt atg cgg cgg atc cga aaa gaa aac gtt gat gcc     336
Ala Val Ser Leu Val Met Arg Arg Ile Arg Lys Glu Asn Val Asp Ala
            100                 105                 110

ggt gaa cgt gca aaa cag gct cta gcg ttc gaa cgc act gat ttc gac 384
Gly Glu Arg Ala Lys Gln Ala Leu Ala Phe Glu Arg Thr Asp Phe Asp
        115                 120                 125

cag gtt cgt tca ctc atg gaa aat agc gat cgc tgc cag gat ata cgt 432
Gln Val Arg Ser Leu Met Glu Asn Ser Asp Arg Cys Gln Asp Ile Arg
    130                 135                 140

aat ctg gca ttt ctg ggg att gct tat aac acc ctg tta cgt ata gcc 480
Asn Leu Ala Phe Leu Gly Ile Ala Tyr Asn Thr Leu Leu Arg Ile Ala
145                 150                 155                 160

gaa att gcc agg atc agg gtt aaa gat atc tca cgt act gac ggt ggg 528
Glu Ile Ala Arg Ile Arg Val Lys Asp Ile Ser Arg Thr Asp Gly Gly
                165                 170                 175

aga atg tta atc cat att ggc aga acg aaa acg ctg gtt agc acc gca 576
Arg Met Leu Ile His Ile Gly Arg Thr Lys Thr Leu Val Ser Thr Ala
            180                 185                 190

ggt gta gag aag gca ctt agc ctg ggg gta act aaa ctg gtc gag cga 624
Gly Val Glu Lys Ala Leu Ser Leu Gly Val Thr Lys Leu Val Glu Arg
        195                 200                 205

tgg att tcc gtc tct ggt gta gct gat gat ccg aat aac tac ctg ttt 672
Trp Ile Ser Val Ser Gly Val Ala Asp Asp Pro Asn Asn Tyr Leu Phe
    210                 215                 220

tgc cgg gtc aga aaa aat ggt gtt gcc gcg cca tct gcc acc agc cag 720
Cys Arg Val Arg Lys Asn Gly Val Ala Ala Pro Ser Ala Thr Ser Gln
225                 230                 235                 240

cta tca act cgc gcc ctg gaa ggg att ttt gaa gca act cat cga ttg 768
Leu Ser Thr Arg Ala Leu Glu Gly Ile Phe Glu Ala Thr His Arg Leu
                245                 250                 255

att tac ggc gct aag gat gac tct ggt cag aga tac ctg gcc tgg tct 816
Ile Tyr Gly Ala Lys Asp Asp Ser Gly Gln Arg Tyr Leu Ala Trp Ser
            260                 265                 270

gga cac agt gcc cgt gtc gga gcc gcg cga gat atg gcc cgc gct gga 864
Gly His Ser Ala Arg Val Gly Ala Ala Arg Asp Met Ala Arg Ala Gly
        275                 280                 285

gtt tca ata ccg gag atc atg caa gct ggt ggc tgg acc aat gta aat 912
Val Ser Ile Pro Glu Ile Met Gln Ala Gly Gly Trp Thr Asn Val Asn
    290                 295                 300

att gtc atg aac tat atc cgt aac ctg gat agt gaa aca ggg gca atg 960
Ile Val Met Asn Tyr Ile Arg Asn Leu Asp Ser Glu Thr Gly Ala Met
305                 310                 315                 320

gcg cgc ctg ctg gaa gat ggc gat ggc gcc tca cca ggt caa gac ata 1008
Ala Arg Leu Leu Glu Asp Gly Asp Gly Ala Ser Pro Gly Gln Asp Ile
                325                 330                 335

cag ttg att cca cca ctg atc aac ctg tta atg agc att gaa cca gat 1056
Gln Leu Ile Pro Pro Leu Ile Asn Leu Leu Met Ser Ile Glu Pro Asp
            340                 345                 350

gtg atc tat gca gga cat gac aac aca aaa cct gac acc tcc agt tct    1104
Val Ile Tyr Ala Gly His Asp Asn Thr Lys Pro Asp Thr Ser Ser Ser
        355                 360                 365

ttg ctg aca agt ctt aat caa cta ggc gag agg caa ctt ctt tca gta    1152
Leu Leu Thr Ser Leu Asn Gln Leu Gly Glu Arg Gln Leu Leu Ser Val
    370                 375                 380

gtc aag tgg tct aaa tca ttg cca ggt ttt cga aac tta cat att gat    1200
Val Lys Trp Ser Lys Ser Leu Pro Gly Phe Arg Asn Leu His Ile Asp
385                 390                 395                 400

gac cag ata act ctc att cag tat tct tgg atg agc tta atg gtg ttt    1248
Asp Gln Ile Thr Leu Ile Gln Tyr Ser Trp Met Ser Leu Met Val Phe
                405                 410                 415

ggt cta gga tgg aga tcc tac aaa cac gtc agt ggg cag atg ctg tat    1296
Gly Leu Gly Trp Arg Ser Tyr Lys His Val Ser Gly Gln Met Leu Tyr
            420                 425                 430

ttt gca cct gat cta ata cta aat gaa cag cgg atg aaa gaa tca tca    1344
Phe Ala Pro Asp Leu Ile Leu Asn Glu Gln Arg Met Lys Glu Ser Ser
        435                 440                 445

ttc tat tca tta tgc ctt acc atg tgg cag atc cca cag gag ttt gtc    1392
Phe Tyr Ser Leu Cys Leu Thr Met Trp Gln Ile Pro Gln Glu Phe Val
    450                 455                 460

aag ctt caa gtt agc caa gaa gag ttc ctc tgt atg aaa gta ttg tta    1440
Lys Leu Gln Val Ser Gln Glu Glu Phe Leu Cys Met Lys Val Leu Leu
465                 470                 475                 480

ctt ctt aat aca att cct ttg gaa ggg cta cga agt caa acc cag ttt    1488
Leu Leu Asn Thr Ile Pro Leu Glu Gly Leu Arg Ser Gln Thr Gln Phe
                485                 490                 495

gag gag atg agg tca agc tac att aga gag ctc atc aag gca att ggt    1536
Glu Glu Met Arg Ser Ser Tyr Ile Arg Glu Leu Ile Lys Ala Ile Gly
            500                 505                 510

ttg agg caa aaa gga gtt gtg tcg agc tca cag cgt ttc tat caa ctt    1584
Leu Arg Gln Lys Gly Val Val Ser Ser Ser Gln Arg Phe Tyr Gln Leu
        515                 520                 525

aca aaa ctt ctt gat aac ttg cat gat ctt gtc aaa caa ctt cat ctg    1632
Thr Lys Leu Leu Asp Asn Leu His Asp Leu Val Lys Gln Leu His Leu
    530                 535                 540

tac tgc ttg aat aca ttt atc cag tcc cgg gca ctg agt gtt gaa ttt    1680
Tyr Cys Leu Asn Thr Phe Ile Gln Ser Arg Ala Leu Ser Val Glu Phe
545                 550                 555                 560

cca gaa atg atg tct gaa gtt att gct atg cat gcg tac gga tag        1725
Pro Glu Met Met Ser Glu Val Ile Ala Met His Ala Tyr Gly
                565                 570

<210> 6
<211> 574
<212> PRT
<213> Künstliche Sequenz

<223> Beschreibung der künstlichen Sequenz:
      Fusionsprotein Bacteriophage
      P1-Cre19V336A-PR676-914

<400> 6
Met Gly Ala Thr Ser Asp Glu Val Arg Lys Asn Leu Met Asp Met Phe
1               5                   10                  15

Arg Asp Arg Gln Ala Phe Ser Glu His Thr Trp Lys Met Leu Leu Ser
            20                  25                  30

Val Cys Arg Ser Trp Ala Ala Trp Cys Lys Leu Asn Asn Arg Lys Trp
        35                  40                  45

Phe Pro Ala Glu Pro Glu Asp Val Arg Asp Tyr Leu Leu Tyr Leu Gln
    50                  55                  60

Ala Arg Gly Leu Ala Val Lys Thr Ile Gln Gln His Leu Gly Gln Leu
65                  70                  75                  80

Asn Met Leu His Arg Arg Ser Gly Leu Pro Arg Pro Ser Asp Ser Asn
                85                  90                  95

Ala Val Ser Leu Val Met Arg Arg Ile Arg Lys Glu Asn Val Asp Ala
            100                 105                 110

Gly Glu Arg Ala Lys Gln Ala Leu Ala Phe Glu Arg Thr Asp Phe Asp
        115                 120                 125

Gln Val Arg Ser Leu Met Glu Asn Ser Asp Arg Cys Gln Asp Ile Arg
    130                 135                 140

Asn Leu Ala Phe Leu Gly Ile Ala Tyr Asn Thr Leu Leu Arg Ile Ala
145                 150                 155                 160

Glu Ile Ala Arg Ile Arg Val Lys Asp Ile Ser Arg Thr Asp Gly Gly
                165                 170                 175

Arg Met Leu Ile His Ile Gly Arg Thr Lys Thr Leu Val Ser Thr Ala
            180                 185                 190

Gly Val Glu Lys Ala Leu Ser Leu Gly Val Thr Lys Leu Val Glu Arg
        195                 200                 205

Trp Ile Ser Val Ser Gly Val Ala Asp Asp Pro Asn Asn Tyr Leu Phe
    210                 215                 220

Cys Arg Val Arg Lys Asn Gly Val Ala Ala Pro Ser Ala Thr Ser Gln
225                 230                 235                 240

Leu Ser Thr Arg Ala Leu Glu Gly Ile Phe Glu Ala Thr His Arg Leu
                245                 250                 255

Ile Tyr Gly Ala Lys Asp Asp Ser Gly Gln Arg Tyr Leu Ala Trp Ser
            260                 265                 270

Gly His Ser Ala Arg Val Gly Ala Ala Arg Asp Met Ala Arg Ala Gly
        275                 280                 285

Val Ser Ile Pro Glu Ile Met Gln Ala Gly Gly Trp Thr Asn Val Asn
    290                 295                 300

Ile Val Met Asn Tyr Ile Arg Asn Leu Asp Ser Glu Thr Gly Ala Met
305                 310                 315                 320

Ala Arg Leu Leu Glu Asp Gly Asp Gly Ala Ser Pro Gly Gln Asp Ile
                325                 330                 335

Gln Leu Ile Pro Pro Leu Ile Asn Leu Leu Met Ser Ile Glu Pro Asp
            340                 345                 350

Val Ile Tyr Ala Gly His Asp Asn Thr Lys Pro Asp Thr Ser Ser Ser
        355                 360                 365

Leu Leu Thr Ser Leu Asn Gln Leu Gly Glu Arg Gln Leu Leu Ser Val
    370                 375                 380

Val Lys Trp Ser Lys Ser Leu Pro Gly Phe Arg Asn Leu His Ile Asp
385                 390                 395                 400

Asp Gln Ile Thr Leu Ile Gln Tyr Ser Trp Met Ser Leu Met Val Phe
                405                 410                 415

Gly Leu Gly Trp Arg Ser Tyr Lys His Val Ser Gly Gln Met Leu Tyr
            420                 425                 430

Phe Ala Pro Asp Leu Ile Leu Asn Glu Gln Arg Met Lys Glu Ser Ser
        435                 440                 445

Phe Tyr Ser Leu Cys Leu Thr Met Trp Gln Ile Pro Gln Glu Phe Val
    450                 455                 460

Lys Leu Gln Val Ser Gln Glu Glu Phe Leu Cys Met Lys Val Leu Leu
465                 470                 475                 480

Leu Leu Asn Thr Ile Pro Leu Glu Gly Leu Arg Ser Gln Thr Gln Phe
                485                 490                 495

Glu Glu Met Arg Ser Ser Tyr Ile Arg Glu Leu Ile Lys Ala Ile Gly
            500                 505                 510

Leu Arg Gln Lys Gly Val Val Ser Ser Ser Gln Arg Phe Tyr Gln Leu
        515                 520                 525

Thr Lys Leu Leu Asp Asn Leu His Asp Leu Val Lys Gln Leu His Leu
    530                 535                 540

Tyr Cys Leu Asn Thr Phe Ile Gln Ser Arg Ala Leu Ser Val Glu Phe
545                 550                 555                 560

Pro Glu Met Met Ser Glu Val Ile Ala Met His Ala Tyr Gly
                565                 570


<210> 7
<211> 1725
<212> DNA
<213> Künstliche Sequenz

<220>
<223> Beschreibung der künstlichen Sequenz:
      Fusionsprotein hCre19V336A-PR676-914

<220>
<221> CDS
<222> (1)..(1722)

<400> 7
atg ggt gcc acc tct gat gaa gtc agg aag aac ctg atg gac atg ttc     48
Met Gly Ala Thr Ser Asp Glu Val Arg Lys Asn Leu Met Asp Met Phe
1               5                   10                  15

agg gac agg cag gcc ttc tct gaa cac acc tgg aag atg ctc ctg tct     96
Arg Asp Arg Gln Ala Phe Ser Glu His Thr Trp Lys Met Leu Leu Ser
            20                  25                  30

gtg tgc aga tcc tgg gct gcc tgg tgc aag ctg aac aac agg aaa tgg     144
Val Cys Arg Ser Trp Ala Ala Trp Cys Lys Leu Asn Asn Arg Lys Trp
        35                  40                  45

ttc cct gct gaa cct gag gat gtg agg gac tac ctc ctg tac ctg caa     192
Phe Pro Ala Glu Pro Glu Asp Val Arg Asp Tyr Leu Leu Tyr Leu Gln
    50                  55                  60

gcc aga ggc ctg gct gtg aag acc atc caa cag cac ctg ggc cag ctc     240
Ala Arg Gly Leu Ala Val Lys Thr Ile Gln Gln His Leu Gly Gln Leu
65                  70                  75                  80

aac atg ctg cac agg aga tct ggc ctg cct cgc cct tct gac tcc aat     288
Asn Met Leu His Arg Arg Ser Gly Leu Pro Arg Pro Ser Asp Ser Asn
                85                  90                  95

gct gtg tcc ctg gtg atg agg aga atc aga aag gag aat gtg gat gct     336
Ala Val Ser Leu Val Met Arg Arg Ile Arg Lys Glu Asn Val Asp Ala
            100                 105                 110

ggg gag aga gcc aag cag gcc ctg gcc ttt gaa cgc act gac ttt gac     384
Gly Glu Arg Ala Lys Gln Ala Leu Ala Phe Glu Arg Thr Asp Phe Asp
        115                 120                 125

caa gtc aga tcc ctg atg gag aac tct gac aga tgc cag gac atc agg     432
Gln Val Arg Ser Leu Met Glu Asn Ser Asp Arg Cys Gln Asp Ile Arg
    130                 135                 140

aac ctg gcc ttc ctg ggc att gcc tac aac acc ctg ctg cgc att gcc     480
Asn Leu Ala Phe Leu Gly Ile Ala Tyr Asn Thr Leu Leu Arg Ile Ala
145                 150                 155                 160

gaa att gcc aga atc aga gtg aag gac atc tcc cgc acc gat ggt ggg     528
Glu Ile Ala Arg Ile Arg Val Lys Asp Ile Ser Arg Thr Asp Gly Gly
                165                 170                 175

aga atg ctg atc cac att ggc agg acc aag acc ctg gtg tcc aca gct     576
Arg Met Leu Ile His Ile Gly Arg Thr Lys Thr Leu Val Ser Thr Ala
            180                 185                 190

ggt gtg gag aag gcc ctg tcc ctg ggg gtt acc aag ctg gtg gag aga     624
Gly Val Glu Lys Ala Leu Ser Leu Gly Val Thr Lys Leu Val Glu Arg
        195                 200                 205

tgg atc tct gtg tct ggt gtg gct gat gac ccc aac aac tac ctg ttc     672
Trp Ile Ser Val Ser Gly Val Ala Asp Asp Pro Asn Asn Tyr Leu Phe
    210                 215                 220

tgc cgg gtc aga aag aat ggt gtg gct gcc cct tct gcc acc tcc caa 720
Cys Arg Val Arg Lys Asn Gly Val Ala Ala Pro Ser Ala Thr Ser Gln
225                 230                 235                 240

ctg tcc acc cgg gcc ctg gaa ggg atc ttt gag gcc acc cac cgc ctg 768
Leu Ser Thr Arg Ala Leu Glu Gly Ile Phe Glu Ala Thr His Arg Leu
                245                 250                 255

atc tat ggt gcc aag gat gac tct ggg cag aga tac ctg gcc tgg tct 816
Ile Tyr Gly Ala Lys Asp Asp Ser Gly Gln Arg Tyr Leu Ala Trp Ser
            260                 265                 270

ggc cac tct gcc aga gtg ggt gct gcc agg gac atg gcc agg gct ggt 864
Gly His Ser Ala Arg Val Gly Ala Ala Arg Asp Met Ala Arg Ala Gly
        275                 280                 285

gtg tcc atc cct gaa atc atg cag gct ggt ggc tgg acc aat gtg aac 912
Val Ser Ile Pro Glu Ile Met Gln Ala Gly Gly Trp Thr Asn Val Asn
    290                 295                 300

att gtg atg aac tac atc aga aac ctg gac tct gag act ggg gcc atg 960
Ile Val Met Asn Tyr Ile Arg Asn Leu Asp Ser Glu Thr Gly Ala Met
305                 310                 315                 320

gcg agg ctg ctc gag gat ggg gac ggc gcc tca cca ggt caa gac ata 1008
Ala Arg Leu Leu Glu Asp Gly Asp Gly Ala Ser Pro Gly Gln Asp Ile
                325                 330                 335

cag ttg att cca cca ctg atc aac ctg tta atg agc att gaa cca gat 1056
Gln Leu Ile Pro Pro Leu Ile Asn Leu Leu Met Ser Ile Glu Pro Asp
            340                 345                 350

gtg atc tat gca gga cat gac aac aca aaa cct gac acc tcc agt tct 1104
Val Ile Tyr Ala Gly His Asp Asn Thr Lys Pro Asp Thr Ser Ser Ser
        355                 360                 365

ttg ctg aca agt ctt aat caa cta ggc gag agg caa ctt ctt tca gta 1152
Leu Leu Thr Ser Leu Asn Gln Leu Gly Glu Arg Gln Leu Leu Ser Val
    370                 375                 380

gtc aag tgg tct aaa tca ttg cca ggt ttt cga aac tta cat att gat 1200
Val Lys Trp Ser Lys Ser Leu Pro Gly Phe Arg Asn Leu His Ile Asp
385                 390                 395                 400

gac cag ata act ctc att cag tat tct tgg atg agc tta atg gtg ttt 1248
Asp Gln Ile Thr Leu Ile Gln Tyr Ser Trp Met Ser Leu Met Val Phe
                405                 410                 415

ggt cta gga tgg aga tcc tac aaa cac gtc agt ggg cag atg ctg tat 1296
Gly Leu Gly Trp Arg Ser Tyr Lys His Val Ser Gly Gln Met Leu Tyr
            420                 425                 430

ttt gca cct gat cta ata cta aat gaa cag cgg atg aaa gaa tca tca 1344
Phe Ala Pro Asp Leu Ile Leu Asn Glu Gln Arg Met Lys Glu Ser Ser
        435                 440                 445

ttc tat tca tta tgc ctt acc atg tgg cag atc cca cag gag ttt gtc 1392
Phe Tyr Ser Leu Cys Leu Thr Met Trp Gln Ile Pro Gln Glu Phe Val
    450                 455                 460

aag ctt caa gtt agc caa gaa gag ttc ctc tgt atg aaa gta ttg tta    1440
Lys Leu Gln Val Ser Gln Glu Glu Phe Leu Cys Met Lys Val Leu Leu
465                 470                 475                 480

ctt ctt aat aca att cct ttg gaa ggg cta cga agt caa acc cag ttt    1488
Leu Leu Asn Thr Ile Pro Leu Glu Gly Leu Arg Ser Gln Thr Gln Phe
                485                 490                 495

gag gag atg agg tca agc tac att aga gag ctc atc aag gca att ggt    1536
Glu Glu Met Arg Ser Ser Tyr Ile Arg Glu Leu Ile Lys Ala Ile Gly
            500                 505                 510

ttg agg caa aaa gga gtt gtg tcg agc tca cag cgt ttc tat caa ctt    1584
Leu Arg Gln Lys Gly Val Val Ser Ser Ser Gln Arg Phe Tyr Gln Leu
        515                 520                 525

aca aaa ctt ctt gat aac ttg cat gat ctt gtc aaa caa ctt cat ctg    1632
Thr Lys Leu Leu Asp Asn Leu His Asp Leu Val Lys Gln Leu His Leu
    530                 535                 540

tac tgc ttg aat aca ttt atc cag tcc cgg gca ctg agt gtt gaa ttt    1680
Tyr Cys Leu Asn Thr Phe Ile Gln Ser Arg Ala Leu Ser Val Glu Phe
545                 550                 555                 560

cca gaa atg atg tct gaa gtt att gct atg cat gcg tac gga tag        1725
Pro Glu Met Met Ser Glu Val Ile Ala Met His Ala Tyr Gly
                565                 570


<210> 8
<211> 574
<212> PRT
<213> Künstliche Sequenz
<223> Beschreibung der künstlichen Sequenz:
      Fusionsprotein hCre19V336A-PR676-914

<400> 8
Met Gly Ala Thr Ser Asp Glu Val Arg Lys Asn Leu Met Asp Met Phe
  1               5                  10                  15

Arg Asp Arg Gln Ala Phe Ser Glu His Thr Trp Lys Met Leu Leu Ser
             20                  25                  30

Val Cys Arg Ser Trp Ala Ala Trp Cys Lys Leu Asn Asn Arg Lys Trp
         35                  40                  45

Phe Pro Ala Glu Pro Glu Asp Val Arg Asp Tyr Leu Leu Tyr Leu Gln
     50                  55                  60

Ala Arg Gly Leu Ala Val Lys Thr Ile Gln Gln His Leu Gly Gln Leu
 65                  70                  75                  80

Asn Met Leu His Arg Arg Ser Gly Leu Pro Arg Pro Ser Asp Ser Asn
                 85                  90                  95

Ala Val Ser Leu Val Met Arg Arg Ile Arg Lys Glu Asn Val Asp Ala
            100                 105                 110

Gly Glu Arg Ala Lys Gln Ala Leu Ala Phe Glu Arg Thr Asp Phe Asp
        115                 120                 125

Gln Val Arg Ser Leu Met Glu Asn Ser Asp Arg Cys Gln Asp Ile Arg
    130                 135                 140

Asn Leu Ala Phe Leu Gly Ile Ala Tyr Asn Thr Leu Leu Arg Ile Ala
145                 150                 155                 160

Glu Ile Ala Arg Ile Arg Val Lys Asp Ile Ser Arg Thr Asp Gly Gly
                165                 170                 175

Arg Met Leu Ile His Ile Gly Arg Thr Lys Thr Leu Val Ser Thr Ala
            180                 185                 190

Gly Val Glu Lys Ala Leu Ser Leu Gly Val Thr Lys Leu Val Glu Arg
        195                 200                 205

Trp Ile Ser Val Ser Gly Val Ala Asp Asp Pro Asn Asn Tyr Leu Phe
    210                 215                 220

Cys Arg Val Arg Lys Asn Gly Val Ala Ala Pro Ser Ala Thr Ser Gln
225                 230                 235                 240

Leu Ser Thr Arg Ala Leu Glu Gly Ile Phe Glu Ala Thr His Arg Leu
                245                 250                 255

Ile Tyr Gly Ala Lys Asp Asp Ser Gly Gln Arg Tyr Leu Ala Trp Ser
            260                 265                 270

Gly His Ser Ala Arg Val Gly Ala Ala Arg Asp Met Ala Arg Ala Gly
        275                 280                 285

Val Ser Ile Pro Glu Ile Met Gln Ala Gly Gly Trp Thr Asn Val Asn
    290                 295                 300

Ile Val Met Asn Tyr Ile Arg Asn Leu Asp Ser Glu Thr Gly Ala Met
305                 310                 315                 320

Ala Arg Leu Leu Glu Asp Gly Asp Gly Ala Ser Pro Gly Gln Asp Ile
                325                 330                 335

Gln Leu Ile Pro Pro Leu Ile Asn Leu Leu Met Ser Ile Glu Pro Asp
            340                 345                 350

Val Ile Tyr Ala Gly His Asp Asn Thr Lys Pro Asp Thr Ser Ser Ser
        355                 360                 365

Leu Leu Thr Ser Leu Asn Gln Leu Gly Glu Arg Gln Leu Leu Ser Val
    370                 375                 380

Val Lys Trp Ser Lys Ser Leu Pro Gly Phe Arg Asn Leu His Ile Asp
385                 390                 395                 400

Asp Gln Ile Thr Leu Ile Gln Tyr Ser Trp Met Ser Leu Met Val Phe
                405                 410                 415

Gly Leu Gly Trp Arg Ser Tyr Lys His Val Ser Gly Gln Met Leu Tyr
            420                 425                 430

Phe Ala Pro Asp Leu Ile Leu Asn Glu Gln Arg Met Lys Glu Ser Ser
        435                 440                 445

Phe Tyr Ser Leu Cys Leu Thr Met Trp Gln Ile Pro Gln Glu Phe Val
    450                 455                 460

Lys Leu Gln Val Ser Gln Glu Glu Phe Leu Cys Met Lys Val Leu Leu
465                 470                 475                 480

Leu Leu Asn Thr Ile Pro Leu Glu Gly Leu Arg Ser Gln Thr Gln Phe
                485                 490                 495

Glu Glu Met Arg Ser Ser Tyr Ile Arg Glu Leu Ile Lys Ala Ile Gly
            500                 505                 510

Leu Arg Gln Lys Gly Val Val Ser Ser Ser Gln Arg Phe Tyr Gln Leu
        515                 520                 525

Thr Lys Leu Leu Asp Asn Leu His Asp Leu Val Lys Gln Leu His Leu
    530                 535                 540

Tyr Cys Leu Asn Thr Phe Ile Gln Ser Arg Ala Leu Ser Val Glu Phe
545                 550                 555                 560

Pro Glu Met Met Ser Glu Val Ile Ala Met His Ala Tyr Gly
                565                 570


<210> 9
<211> 1800
<212> DNA
<213> Künstliche Sequenz

<220>
<223> Beschreibung der künstlichen Sequenz:
      Fusionsprotein hCre19V336A-PR650-914

<220>
<221> CDS
<222> (1)..(1797)

<400> 9
atg ggt gcc acc tct gat gaa gtc agg aag aac ctg atg gac atg ttc      48
Met Gly Ala Thr Ser Asp Glu Val Arg Lys Asn Leu Met Asp Met Phe
  1               5                  10                  15

agg gac agg cag gcc ttc tct gaa cac acc tgg aag atg ctc ctg tct      96
Arg Asp Arg Gln Ala Phe Ser Glu His Thr Trp Lys Met Leu Leu Ser
             20                  25                  30

gtg tgc aga tcc tgg gct gcc tgg tgc aag ctg aac aac agg aaa tgg     144
Val Cys Arg Ser Trp Ala Ala Trp Cys Lys Leu Asn Asn Arg Lys Trp
         35                  40                  45

ttc cct gct gaa cct gag gat gtg agg gac tac ctc ctg tac ctg caa     192
Phe Pro Ala Glu Pro Glu Asp Val Arg Asp Tyr Leu Leu Tyr Leu Gln
     50                  55                  60

gcc aga ggc ctg gct gtg aag acc atc caa cag cac ctg ggc cag ctc     240
Ala Arg Gly Leu Ala Val Lys Thr Ile Gln Gln His Leu Gly Gln Leu
 65                  70                  75                  80

aac atg ctg cac agg aga tct ggc ctg cct cgc cct tct gac tcc aat 288
Asn Met Leu His Arg Arg Ser Gly Leu Pro Arg Pro Ser Asp Ser Asn
                85                  90                  95

gct gtg tcc ctg gtg atg agg aga atc aga aag gag aat gtg gat gct 336
Ala Val Ser Leu Val Met Arg Arg Ile Arg Lys Glu Asn Val Asp Ala
            100                 105                 110

ggg gag aga gcc aag cag gcc ctg gcc ttt gaa cgc act gac ttt gac 384
Gly Glu Arg Ala Lys Gln Ala Leu Ala Phe Glu Arg Thr Asp Phe Asp
        115                 120                 125

caa gtc aga tcc ctg atg gag aac tct gac aga tgc cag gac atc agg 432
Gln Val Arg Ser Leu Met Glu Asn Ser Asp Arg Cys Gln Asp Ile Arg
    130                 135                 140

aac ctg gcc ttc ctg ggc att gcc tac aac acc ctg ctg cgc att gcc 480
Asn Leu Ala Phe Leu Gly Ile Ala Tyr Asn Thr Leu Leu Arg Ile Ala
145                 150                 155                 160

gaa att gcc aga atc aga gtg aag gac atc tcc cgc acc gat ggt ggg 528
Glu Ile Ala Arg Ile Arg Val Lys Asp Ile Ser Arg Thr Asp Gly Gly
                165                 170                 175

aga atg ctg atc cac att ggc agg acc aag acc ctg gtg tcc aca gct 576
Arg Met Leu Ile His Ile Gly Arg Thr Lys Thr Leu Val Ser Thr Ala
            180                 185                 190

ggt gtg gag aag gcc ctg tcc ctg ggg gtt acc aag ctg gtg gag aga 624
Gly Val Glu Lys Ala Leu Ser Leu Gly Val Thr Lys Leu Val Glu Arg
        195                 200                 205

tgg atc tct gtg tct ggt gtg gct gat gac ccc aac aac tac ctg ttc 672
Trp Ile Ser Val Ser Gly Val Ala Asp Asp Pro Asn Asn Tyr Leu Phe
    210                 215                 220

tgc cgg gtc aga aag aat ggt gtg gct gcc cct tct gcc acc tcc caa 720
Cys Arg Val Arg Lys Asn Gly Val Ala Ala Pro Ser Ala Thr Ser Gln
225                 230                 235                 240

ctg tcc acc cgg gcc ctg gaa ggg atc ttt gag gcc acc cac cgc ctg 768
Leu Ser Thr Arg Ala Leu Glu Gly Ile Phe Glu Ala Thr His Arg Leu
                245                 250                 255

atc tat ggt gcc aag gat gac tct ggg cag aga tac ctg gcc tgg tct 816
Ile Tyr Gly Ala Lys Asp Asp Ser Gly Gln Arg Tyr Leu Ala Trp Ser
            260                 265                 270

ggc cac tct gcc aga gtg ggt gct gcc agg gac atg gcc agg gct ggt 864
Gly His Ser Ala Arg Val Gly Ala Ala Arg Asp Met Ala Arg Ala Gly
        275                 280                 285

gtg tcc atc cct gaa atc atg cag gct ggt ggc tgg acc aat gtg aac 912
Val Ser Ile Pro Glu Ile Met Gln Ala Gly Gly Trp Thr Asn Val Asn
    290                 295                 300

att gtg atg aac tac atc aga aac ctg gac tct gag act ggg gcc atg 960
Ile Val Met Asn Tyr Ile Arg Asn Leu Asp Ser Glu Thr Gly Ala Met
305                 310                 315                 320

gcg agg ctg ctc gag gat ggg gac ggc gcc ctg gat gct gtt gct ctc     1008
Ala Arg Leu Leu Glu Asp Gly Asp Gly Ala Leu Asp Ala Val Ala Leu
                325                 330                 335

cca cag cca gtg ggc gtt cca aat gaa agc caa gcc cta agc cag aga     1056
Pro Gln Pro Val Gly Val Pro Asn Glu Ser Gln Ala Leu Ser Gln Arg
            340                 345                 350

ttc act ttt tca cca ggt caa gac ata cag ttg att cca cca ctg atc     1104
Phe Thr Phe Ser Pro Gly Gln Asp Ile Gln Leu Ile Pro Pro Leu Ile
        355                 360                 365

aac ctg tta atg agc att gaa cca gat gtg atc tat gca gga cat gac     1152
Asn Leu Leu Met Ser Ile Glu Pro Asp Val Ile Tyr Ala Gly His Asp
    370                 375                 380

aac aca aaa cct gac acc tcc agt tct ttg ctg aca agt ctt aat caa     1200
Asn Thr Lys Pro Asp Thr Ser Ser Ser Leu Leu Thr Ser Leu Asn Gln
385                 390                 395                 400

cta ggc gag agg caa ctt ctt tca gta gtc aag tgg tct aaa tca ttg     1248
Leu Gly Glu Arg Gln Leu Leu Ser Val Val Lys Trp Ser Lys Ser Leu
                405                 410                 415

cca ggt ttt cga aac tta cat att gat gac cag ata act ctc att cag     1296
Pro Gly Phe Arg Asn Leu His Ile Asp Asp Gln Ile Thr Leu Ile Gln
            420                 425                 430

tat tct tgg atg agc tta atg gtg ttt ggt cta gga tgg aga tcc tac     1344
Tyr Ser Trp Met Ser Leu Met Val Phe Gly Leu Gly Trp Arg Ser Tyr
        435                 440                 445

aaa cac gtc agt ggg cag atg ctg tat ttt gca cct gat cta ata cta     1392
Lys His Val Ser Gly Gln Met Leu Tyr Phe Ala Pro Asp Leu Ile Leu
    450                 455                 460

aat gaa cag cgg atg aaa gaa tca tca ttc tat tca tta tgc ctt acc     1440
Asn Glu Gln Arg Met Lys Glu Ser Ser Phe Tyr Ser Leu Cys Leu Thr
465                 470                 475                 480

atg tgg cag atc cca cag gag ttt gtc aag ctt caa gtt agc caa gaa     1488
Met Trp Gln Ile Pro Gln Glu Phe Val Lys Leu Gln Val Ser Gln Glu
                485                 490                 495

gag ttc ctc tgt atg aaa gta ttg tta ctt ctt aat aca att cct ttg     1536
Glu Phe Leu Cys Met Lys Val Leu Leu Leu Leu Asn Thr Ile Pro Leu
            500                 505                 510

gaa ggg cta cga agt caa acc cag ttt gag gag atg agg tca agc tac     1584
Glu Gly Leu Arg Ser Gln Thr Gln Phe Glu Glu Met Arg Ser Ser Tyr
        515                 520                 525

att aga gag ctc atc aag gca att ggt ttg agg caa aaa gga gtt gtg     1632
Ile Arg Glu Leu Ile Lys Ala Ile Gly Leu Arg Gln Lys Gly Val Val
    530                 535                 540

tcg agc tca cag cgt ttc tat caa ctt aca aaa ctt ctt gat aac ttg     1680
Ser Ser Ser Gln Arg Phe Tyr Gln Leu Thr Lys Leu Leu Asp Asn Leu
545                 550                 555                 560

cat gat ctt gtc aaa caa ctt cat ctg tac tgc ttg aat aca ttt atc     1728
His Asp Leu Val Lys Gln Leu His Leu Tyr Cys Leu Asn Thr Phe Ile
                565                 570                 575

cag tcc cgg gca ctg agt gtt gaa ttt cca gaa atg atg tct gaa gtt     1776
Gln Ser Arg Ala Leu Ser Val Glu Phe Pro Glu Met Met Ser Glu Val
            580                 585                 590

att gct atg cat gcg tac gga tag                                     1800
Ile Ala Met His Ala Tyr Gly
        595

<210> 10
<211> 599
<212> PRT
<213> Künstliche Sequenz
<223> Beschreibung der künstlichen Sequenz:
      Fusionsprotein hCre19V336A-PR650-914

<400> 10
Met Gly Ala Thr Ser Asp Glu Val Arg Lys Asn Leu Met Asp Met Phe
  1               5                  10                  15

Arg Asp Arg Gln Ala Phe Ser Glu His Thr Trp Lys Met Leu Leu Ser
             20                  25                  30

Val Cys Arg Ser Trp Ala Ala Trp Cys Lys Leu Asn Asn Arg Lys Trp
         35                  40                  45

Phe Pro Ala Glu Pro Glu Asp Val Arg Asp Tyr Leu Leu Tyr Leu Gln
     50                  55                  60

Ala Arg Gly Leu Ala Val Lys Thr Ile Gln Gln His Leu Gly Gln Leu
 65                  70                  75                  80

Asn Met Leu His Arg Arg Ser Gly Leu Pro Arg Pro Ser Asp Ser Asn
                 85                  90                  95

Ala Val Ser Leu Val Met Arg Arg Ile Arg Lys Glu Asn Val Asp Ala
            100                 105                 110

Gly Glu Arg Ala Lys Gln Ala Leu Ala Phe Glu Arg Thr Asp Phe Asp
        115                 120                 125

Gln Val Arg Ser Leu Met Glu Asn Ser Asp Arg Cys Gln Asp Ile Arg
    130                 135                 140

Asn Leu Ala Phe Leu Gly Ile Ala Tyr Asn Thr Leu Leu Arg Ile Ala
145                 150                 155                 160

Glu Ile Ala Arg Ile Arg Val Lys Asp Ile Ser Arg Thr Asp Gly Gly
                165                 170                 175

Arg Met Leu Ile His Ile Gly Arg Thr Lys Thr Leu Val Ser Thr Ala
            180                 185                 190

Gly Val Glu Lys Ala Leu Ser Leu Gly Val Thr Lys Leu Val Glu Arg
        195                 200                 205

Trp Ile Ser Val Ser Gly Val Ala Asp Asp Pro Asn Asn Tyr Leu Phe
    210                 215                 220

Cys Arg Val Arg Lys Asn Gly Val Ala Ala Pro Ser Ala Thr Ser Gln
225                 230                 235                 240

Leu Ser Thr Arg Ala Leu Glu Gly Ile Phe Glu Ala Thr His Arg Leu
                245                 250                 255

Ile Tyr Gly Ala Lys Asp Asp Ser Gly Gln Arg Tyr Leu Ala Trp Ser
            260                 265                 270

Gly His Ser Ala Arg Val Gly Ala Ala Arg Asp Met Ala Arg Ala Gly
        275                 280                 285

Val Ser Ile Pro Glu Ile Met Gln Ala Gly Gly Trp Thr Asn Val Asn
    290                 295                 300

Ile Val Met Asn Tyr Ile Arg Asn Leu Asp Ser Glu Thr Gly Ala Met
305                 310                 315                 320

Ala Arg Leu Leu Glu Asp Gly Asp Gly Ala Leu Asp Ala Val Ala Leu
                325                 330                 335

Pro Gln Pro Val Gly Val Pro Asn Glu Ser Gln Ala Leu Ser Gln Arg
            340                 345                 350

Phe Thr Phe Ser Pro Gly Gln Asp Ile Gln Leu Ile Pro Pro Leu Ile
        355                 360                 365

Asn Leu Leu Met Ser Ile Glu Pro Asp Val Ile Tyr Ala Gly His Asp
    370                 375                 380

Asn Thr Lys Pro Asp Thr Ser Ser Ser Leu Leu Thr Ser Leu Asn Gln
385                 390                 395                 400

Leu Gly Glu Arg Gln Leu Leu Ser Val Val Lys Trp Ser Lys Ser Leu
                405                 410                 415

Pro Gly Phe Arg Asn Leu His Ile Asp Asp Gln Ile Thr Leu Ile Gln
            420                 425                 430

Tyr Ser Trp Met Ser Leu Met Val Phe Gly Leu Gly Trp Arg Ser Tyr
        435                 440                 445

Lys His Val Ser Gly Gln Met Leu Tyr Phe Ala Pro Asp Leu Ile Leu
    450                 455                 460

Asn Glu Gln Arg Met Lys Glu Ser Ser Phe Tyr Ser Leu Cys Leu Thr
465                 470                 475                 480

Met Trp Gln Ile Pro Gln Glu Phe Val Lys Leu Gln Val Ser Gln Glu
                485                 490                 495

Glu Phe Leu Cys Met Lys Val Leu Leu Leu Leu Asn Thr Ile Pro Leu
            500                 505                 510

Glu Gly Leu Arg Ser Gln Thr Gln Phe Glu Glu Met Arg Ser Ser Tyr
        515                 520                 525

Ile Arg Glu Leu Ile Lys Ala Ile Gly Leu Arg Gln Lys Gly Val Val
    530                 535                 540

Ser Ser Ser Gln Arg Phe Tyr Gln Leu Thr Lys Leu Leu Asp Asn Leu
545                 550                 555                 560

His Asp Leu Val Lys Gln Leu His Leu Tyr Cys Leu Asn Thr Phe Ile
                565                 570                 575

Gln Ser Arg Ala Leu Ser Val Glu Phe Pro Glu Met Met Ser Glu Val
            580                 585                 590

Ile Ala Met His Ala Tyr Gly
        595


<210> 11
<211> 81
<212> DNA
<213> Künstliche Sequenz

<220>
<223> Beschreibung der künstlichen Sequenz: 3´-Ende der
      Cre-kodierenden Sequenz von Bacteriophagen P1-Cre

<400> 11
aatgtaaata ttgtcatgaa ctatatccgt aacctggata gtgaaacagg ggcaatggtg 60
cgcctgctgg aagatggcga t                                           81


<210> 12
<211> 81
<212> DNA
<213> Künstliche Sequenz

<220>
<223> Beschreibung der künstlichen Sequenz: 3´-Ende der
      Cre-kodierenden Sequenz von hCre

<400> 12
aatgtgaaca ttgtgatgaa ctacatcaga aacctggact ctgagactgg ggccatggtg 60
aggctgctcg aggatgggga c                                           81


<210> 13
<211> 81
<212> DNA
<213> Künstliche Sequenz

<220>
<223> Beschreibung der künstlichen Sequenz: 3´-Ende der
      Cre-kodierenden Sequenz von P1-CreV336A

<400> 13
aatgtaaata ttgtcatgaa ctatatccgt aacctggata gtgaaacagg ggcaatggcg 60
cgcctgctgg aagatggcga t                                           81


<210> 14
<211> 81
<212> DNA

<213> Künstliche Sequenz

<220>
<223> Beschreibung der künstlichen Sequenz: 3´-Ende der
      Cre-kodierenden Sequenz von hCreV336A

<400> 14
aatgtgaaca ttgtgatgaa ctacatcaga aacctggact ctgagactgg ggccatggcg 60
aggctgctcg aggatgggga c                                           81


<210> 15
<211> 31
<212> DNA
<213> Künstliche Sequenz

<220>
<223> Beschreibung der künstlichen Sequenz: Primer

<400> 15
aaattcgtac gcatcgccat cttccagcag g                                31


<210> 16
<211> 30
<212> DNA
<213> Künstliche Sequenz

<220>
<223> Beschreibung der künstlichen Sequenz: Primer

<400> 16
aaattggcgc catcgccatc ttccagcagg                                  30


<210> 17
<211> 29
<212> DNA
<213> Künstliche Sequenz

<220>
<223> Beschreibung der künstlichen Sequenz: Primer

<400> 17
aatttggcgc cgtccccatc ctcgagcag                                   29


<210> 18
<211> 44
<212> DNA
<213> Künstliche Sequenz

<220>
<223> Beschreibung der künstlichen Sequenz: Primer

<400> 18
aaattggcgc catcgccatc ttccagcagg cgcgccattg cccc                  44


<210> 19
<211> 44

<212> DNA
<213> Künstliche Sequenz

<220>
<223> Beschreibung der künstlichen Sequenz: Primer

<400> 19
aaattggcgc cgtccccatc ctcgagcagc ctcgccatgg cccc 44

<210> 20
<211> 39
<212> DNA
<213> Künstliche Sequenz

<220>
<223> Beschreibung der künstlichen Sequenz: Primer

<400> 20
gggggatcca ccatgggtgc ctccaacctg ctgactgtg 39

<210> 21
<211> 42
<212> DNA
<213> Künstliche Sequenz

<220>
<223> Beschreibung der künstlichen Sequenz: Primer

<400> 21
tttaaggatc caccatgggt gccacgagtg atgaggttcg ca 42

<210> 22
<211> 32
<212> DNA
<213> Künstliche Sequenz

<220>
<223> Beschreibung der künstlichen Sequenz: Primer

<400> 22
tttaacgtac ggcacgagtg atgaggttcg ca 32

<210> 23
<211> 30
<212> DNA
<213> Künstliche Sequenz

<220>
<223> Beschreibung der künstlichen Sequenz: Primer

<400> 23
tttaaggcgc cacgagtgat gaggttcgca 30

## Beschreibung

Die vorliegende Erfindung betrifft eine DNA-Sequenz, die für eine Mutante der Bakteriophagen P1-Rekombinase kodiert, in der eine spezielle kryptische Spleißstelle durch eine die Cre-Proteinsequenz ändernde Basenmutation ausgeschaltet ist, sowie eine DNA-Sequenz, die für ein Fusionsprotein aus der genannten Cre-Mutanten und einer ligandenbindenden Domäne eines Rezeptorproteins kodiert. Die Erfindung betrifft weiterhin Vektoren, Mikroorganismen und transgene Lebewesen, die solch eine DNA-Sequenz enthalten, sowie Cre-Mutanten oder Cre-Fusionsproteine, die von diesen DNA-Sequenzen kodiert werden.

Unter Verwendung ortsspezifischer Rekombinasen können gezielte Veränderungen im Genom von Säugetieren vorgenommen werden. Die Bakteriophagen-P1-Rekombinase Cre erkennt spezifische Basensequenzen (loxP-Stellen) und kann -je nach Lage und Kombination dieser loxP-Stellen im Genom-Gene deletieren, invertieren, translozieren oder auf eine andere gewünschte Weise mutieren (zur Übersicht siehe Rajewsky et al., J. Clin. Invest. 98, 51 - 53 (1996)). Um das Mutationsereignis zu einem beliebigen Zeitpunkt in einem gezielten Gewebe oder Zelltyp der Maus zu induzieren, wurden u.a. posttranslationale Schalter für Cre-Aktivität entwickelt (Schwenk et al., Nucleic Acids Research 26, 1427-1432 (1998)). Dabei handelt es sich um Fusionsproteine aus Cre und der Liganden-bindenden Domäne von Steroid-Rezeptoren (nachfolgend "LBD", z. B. der Progesteron-Rezeptor, Östrogen-Rezeptor oder Glucocorticoid-Rezeptor). Derartige Cre-LBD-Fusionsproteine sind hinsichtlich ihrer Cre-Rekombinase-Funktionalität inaktiv, können aber durch Zugabe der entsprechenden Liganden (posttranslational) aktiviert werden. In Zellkultur bzw. transgenen Tieren wurden Mutanten von LBD getestet, die synthetische gegenüber natürlichen Liganden bevorzugen, damit die Cre-Aktivierung nicht durch endogene Hormone ausgelöst werden kann.

Die Anwendung dieser Technik in transgenen Mäusen unter Verwendung sowohl ubiquitär-aktiver, als auch zelltyp-spezifischer Promotoren ist publiziert worden (z.B. Schwenk et al. (1998); Kellendonk et al., J. Mol. Biol. 285, 175-182 (1999)). Zwar wird in diesen Arbeiten gezeigt, daß die posttranslationale Aktivierung von Cre über LBD prinzipiell funktioniert, das System hat jedoch zwei entscheidende Nachteile:
1) verminderte Aktivität von Cre-LBD in Vergleich zum unfusionierten Cre;
2) Hintergrund-Aktivität von Cre-LBD, d.h. Cre-Rekombinase-Aktivität auch in Abwesenheit von dem Liganden.

Die Hintergrund-Aktivität hat sich bei der Anwendung in vivo als problematisch erwiesen, insbesondere wenn dieses Ereignis sehr früh im Verlauf der Entwicklung eintritt. Dabei entstehen Mosaik-Tiere, in denen in einem Teil der Zellen das Rekombinationsereignis bereits stattgefunden hat, während im anderen Teil der Zellen die posttranslationale Kontrolle noch möglich ist. Wünschenswert wäre die Verfügbarkeit eines besser kontrollierten Cre-Regulationssystems, welches keine (meßbare) Hintergrund-Aktivität aufweist. An diesem Punkt setzt die hier vorgestellte Erfindung an.

Als mögliche Ursachen für die Hintergrundaktivität kommen in Frage:
1) kryptisches Spleißen der Cre-LBD-kodierenden mRNA in der Weise, daß ein trunkiertes Rest-Protein mit unregulierter Cre-Aktivität translatiert werden kann und
2) posttranslationale Degradation durch z.B. endogene Proteasen, die ein unreguliertes Cre prozessieren.

Überraschenderweise wurde gefunden, daß - wenn bestimmte Modifikationen in Cre-kodierenden Sequenzen wie z. B. Cre-LBD-kodierende Sequenzen eingeführt werden - diese unerwünschten Prozesse eliminiert werden:
1) Optimierung der Cre-kodierenden Sequenz hinsichtlich der Ausschaltung kritischer Spleißereignisse
2) Mutation bzw. Verkürzung des Linkerbereichs zwischen Cre und LBD, um die Zugänglichkeit für Proteasen in diesem Bereich zu erschweren.

Die vorliegende Erfindung betrifft somit:
(1) eine DNA-Sequenz, die für eine Mutante der Bakteriophagen-P1-Rekombinase Cre kodiert, in der die kryptische Spleißstelle in der Sequenz ATG GTG CGC, die der Position 1003 - 1011 in der in SEQ ID NO: 1 gezeigten Wildtypsequenz entspricht, durch eine die Cre-Proteinsequenz ändernde Basenmutation ausgeschaltet ist;
(2) eine DNA-Sequenz, die für ein Fusionsprotein aus der Bakteriophagen-P1-Rekombinase Cre und einem weiteren funktionellen Protein kodiert, wobei die die Bakteriophagen-P1-Rekombinase Cre kodierende Sequenz wie in (1) definiert ist;
(3) eine bevorzugte Ausführungsform der unter (2) definierten DNA-Sequenz, wobei das weitere funktionelle Protein eine ligandenbindenden Domäne eines Rezeptorproteins ist;
(4) ein Vektor, enthaltend eine DNA-Sequenz gemäß (1), (2) und/oder (3);
(5) ein Mikroorganismus oder ein transgenes Lebewesen, enthaltend einen Vektor, wie in (4) definiert, und/oder eine DNA-Sequenz, wie in (1), (2) und/oder (3) definiert;
(6) ein Cre-Mutante oder Cre-Fusionsprotein, das von der DNA-Sequenz gemäß (1), (2) und/oder (3) kodiert wird;
(7) Verfahren zur Herstellung einer Cre-Mutante oder eines Cre-Fusionsproteins, wie in (6) definiert, umfassend das Kultivieren eines Mikroorganismusses oder einer Zellstruktur, der/die mit einem Vektor gemäß (4) transformiert oder transfiziert ist; und
(8) Verwendung einer DNA-Sequenz gemäß (2) oder (3) zur Mutagenese und/oder Rekombination von loxP-Stellen enthaltende Zielsequenzen in Lebewesen.

### Figurenbeschreibung

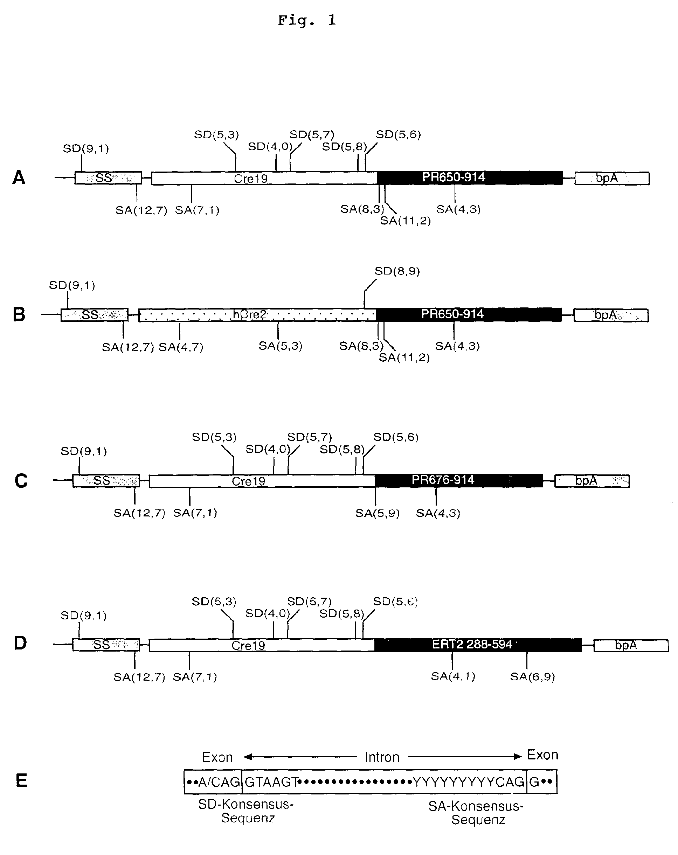

Fig. 1: Spleißmuster ausgewählter Cre-LBD-Konstrukte
   A - D) Dargestellt sind die prä-mRNAs ausgewählter Cre-LBD Konstrukte mit kryptischen Spleiß- Donoren (SD) und Akzeptoren (SA). Die in Klammern angegebenen Werte drücken relative Wahrscheinlichkeiten der Nutzung dieser Stellen aus ('Score'). Der Score gibt an, wie ähnlich die Spleißstellen den Konsensus-Sequenzen (E) sind. Eine 100 %-ige Übereinstimmung zur SA ergibt einen Score von 14,2 und der Mittelwert für konstitutive Exons ist 7,9. Eine 100 %-ige Übereinstimmung mit der SD ergibt einen Score von 12,6 und der Mittelwert für konstitutive Exons ist 8,1. Eingezeichnet sind nur SD und SA, deren Score über 4 liegt. Die Scores werden berechnet mit Hilfe des Computerprogramms "splice site score calculation" (http://www2.imcb.osaka-u.ac.jp/splice/score.html). Verwendete Abkürzungen: SS, synthetisches Spleißsubstrat (Choi et al., Mol. Cell. Biol., 11, 3070 - 3074 (1991)); Cre19, Cre beginnend mit AS 19; hCre2, humanisiertes Cre beginnend mit AS 2; PR bzw. ER, LBD des Progesteron- bzw. Östrogenrezeptors, Zahlen geben AS-Position an; ERT2, LBD des Östrogenrezeptors mit den AS-Substitutionen G400V, M543A, L544A (Feil et al., Biochem. Biophys. Res. Commun. 237, 752 - 757 (1997)). bpA, Polyadenylierungssignal des Wachstumshormons vom Rind; Y, entweder C oder T.

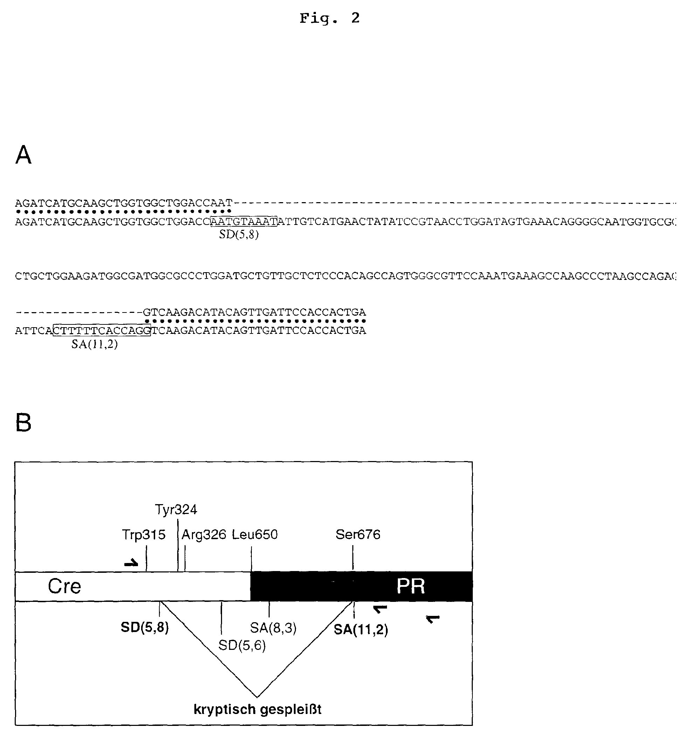

Fig. 2: Ein über RT-PCR nachgewiesenes kryptisches Spleißereignis eines P1-CrePR650-914-kodierenden Konstrukts
   Dargestellt ist der Fusionsbereich von Cre19PR650-914.
   A) Sequenzvergleich der kryptisch gespleißten Sequenz (oben) mit der entsprechenden ungespleißten Sequenz (unten).
   B) Schematische Darstellung des kryptischen Spleiß-Ereignisses. Wichtige AS in diesem Bereich sind eingezeichnet. Die Pfeile zeigen den Verlauf der PCR an: nach der RT-Reaktion wurde die Sequenz mit dem 5'-Primer und dem äußerem 3'-Primer amplifiziert. Nach der Isolation aus dem Agarosegel wurde eine semi-nested PCR mit dem 5'-Primer und dem weiter innen liegenden 3'-Primer vollzogen.

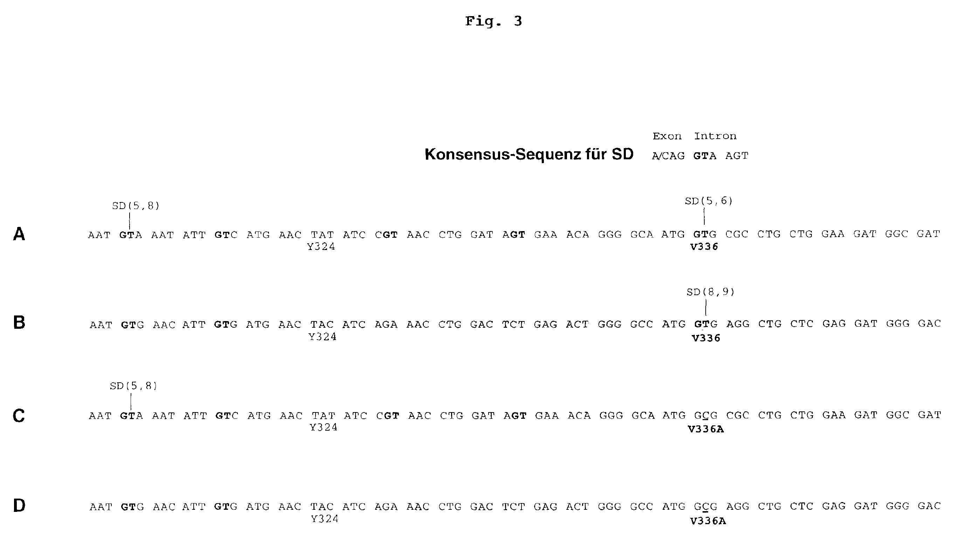

Fig. 3: Sequenzvergleich der 3'-Enden der verschiedenen Cre-kodierenden Sequenzen mit dem Konsensus für Spleiß-Donoren
   Ausschnitt aus dem 3'-Ende der Cre-kodierenden Sequenz von
   A) P1-Cre
   B) hCre
   C) P1-CreV336A und
   D) hCreV336A.
   Gezeigt ist der Bereich der Cre-Sequenz, wo die AS-Substitution eingeführt wurde. Die Basensequenz um V336 im P1-Cre zeigt eine hohe Homologie zum dargestellten Konsensus einer Spleiß-Donor-Stelle. Die Übereinstimmung nimmt im hCre noch zu, damit besteht ein höheres Risiko von kryptischem Spleißen an dieser Stelle. In den mutierten Sequenzen von P1-Cre und hCre wurde das Kodon 336 (Numerierung bezogen auf die in SEQ ID NO: 1 gezeigte Wildtypsequenz) von GTG (Val) nach GCG (Ala) ausgetauscht; durch diesen Austausch ist kein Spleißen mehr möglich, da die Sequenz GTN als essentiell angesehen werden kann. Der Basenaustausch von T nach C ist an Position 1007, bezogen auf die Basensequenz des Wildtyp-Cre (SEQ. ID. NO:1, nach Sternberg et al., 1986)

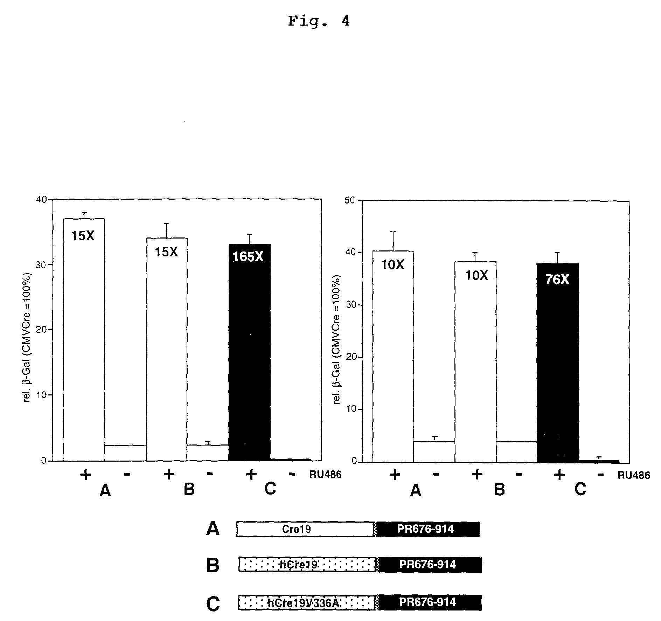

Fig. 4: Aktivitätstest von Cre19PR676-914, hCre19PR676-914 und hCre19V336APR676-914 gemäß Beispiel 6
   Die Balken geben Mittelwerte aus jeweils drei verschiedenen Transfektionen mit Standardabweichung wieder. Weiße Balken stehen für prozentuale Aktivitäten der unmutierten Cre19PR676-914 bzw. hCre19PR676-914, schwarze Balken stehen für mutiertes hCre19V336APR676-914. Die Zahlen in den Balken geben die Induzierbarkeit des betreffenden Konstrukts an, die durch Division der prozentualen Aktivität im induzierten Zustand durch den Hintergrund errechnet wird. "+": 100 nM RU486 in Medium, "-": kein Induktor im Medium.

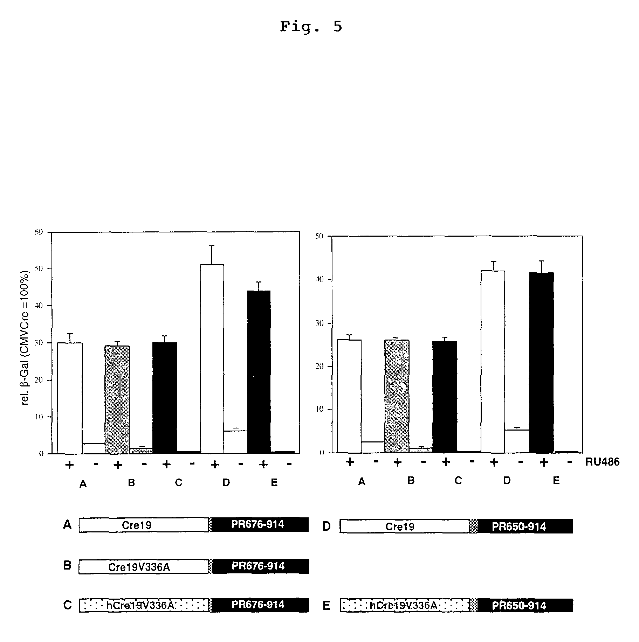

Fig. 5: Aktivitätstest von mutiertem P1-Cre im Vergleich zu mutiertem hCre in Fusion mit PR
   Das Experiment wurde wie in Beispiel 6 beschrieben durchgeführt. Dargestellt sind zwei unabhängige Transfektionsrunden, deren Balken für Mittelwerte aus drei verschiedenen Transfektionen mit Standardabweichung stehen. Weiße Balken stehen für prozentuale Aktivitäten der Cre19-LBD-Konstrukte relativ zu CMV-Cre, graue Balken stehen für Cre19V336APR676-914, schwarze Balken stehen für hCrel9V336A-LBD-Konstrukte. "+": 100 nM RU486 in Medium; "-": kein Induktor in Medium.

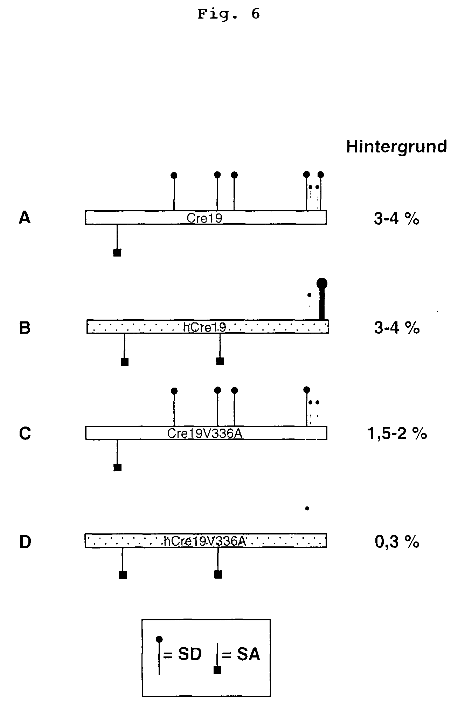

Fig. 6: Schematischer Vergleich der kryptischen Spleißmuster der verwendeten Cre-Sequenzen und Auswirkungen auf die Cre-Aktivität in Abwesenheit des Induktors
   Die angegeben Prozentwerte beziehen sich auf den Vergleich der gezeigten Cre-Konstrukte in Fusion mit PR676-914 relativ zu unfusioniertem Cre (=100 %).

Die vorliegende Erfindung wird nachfolgend näher beschrieben.

Um das Ausmaß kryptischer Spleißvorgänge in Cre-Fusionsproteinen abschätzen zu können, wurden über Computeranalysen Muster potentieller Spleißstellen erstellt (Fig. 1). Innerhalb der Cre-kodierenden Sequenz vom Bakteriophagen P1 (fortan kurz P1) tritt ein relativ starker Akzeptor am 5'-terminal auf, sowie fünf Donoren mittlerer Stärke in der 3'-Hälfte (Fig. 1 A). In der LBD-kodierenden Sequenz des Progesteron-Rezeptors (Nukleotide 1948 bis 2742 von SEQ ID NO:3, die für AS650 bis 914 des Wildtyp-Rezeptors kodieren, nachfolgend "PR") treten zwei Akzeptoren mittlerer, sowie ein SA mit extrem hoher Stärke auf (Fig. 1A-B). In der entsprechenden Östrogen-Rezeptor-Sequenz (nachfolgend "ER") liefert die Analyse nur zwei SA mit mittlerer Stärke (Fig. 1D). Eine oder mehrere der am 3'-Ende von P1-Cre liegenden Donor-Sequenzen könnten mit einem der Akzeptoren in PR bzw. ER ein Intron bilden. Durch Spleißen dieses Introns würde ein vorzeitiger Abbruch der Translation infolge der Verschiebung des Leserasters generiert, resultierend in ein verkürztes, möglicherweise konstitutiv aktives Cre-Protein ohne den regulatorischen Anteil der LBD. Es konnte über RT-PCR nachgewiesen werden, daß ein derartiges kryptisches Spleißen in eukaryontischen Zellen tatsächlich auftritt (siehe Fig. 2). Die Funktionalität der SA (11,2) im PR in physiologischem Kontext wurde von Balleine et al., J. Clin. End. Meta. 84, 1370-1377 (1999) publiziert. In dieser Publikation wird ein alternativ gespleißtes Transkript des humanen Progesteronrezeptors beschrieben, das in gehäuftem Maße in Brustkrebstumoren auftritt. Normalerweise ist es von geringer Bedeutung, wenn ein sehr geringer Anteil transgener Transkripte kryptisch gespleißt wird. Doch im Falle von Cre-LBD-Transkripten kann auch nur ein verschwindender Teil kryptischer Spleißprodukte das Auftreten der beobachteten Cre-Hintergrundaktivität erklären.

Durch Angleichung des Kodon-usage wird die Cre-Sequenz (F.Stewart, unveröffentlichte Ergebnisse) diesbezüglich zwar verbessert, da viele kryptische Spleißstellen durch stille Mutationen eliminiert werden (Fig. 1A und B); eine Spleiß-Donor-Stelle direkt am 3'-Ende wird jedoch nicht eliminiert - sondern im Gegenteil eher verstärkt durch Kodon-Optimierung ('SD (8,9)' in Abb 1B). Wenn man die Basensequenz an dieser Position betrachtet (Fig. 3), wird offensichtlich, daß durch stille Mutation keine Eliminierung dieser SD möglich ist: jedes für Valin kodierende Kodon (GTN) an AS-Position 336 des Wildtyps beinhaltet die für das Spleiß-Ereignis zwei kritischen Basen GT. Es wurde eine Mutation eingeführt, welche die kritische GT-Sequenz zerstört (GTG nach GCG, Austausch an Basenposition 1007, bezogen auf die kodierende Basensequenz der Wildtyp-Cre-Sequenz, vgl. Sternberg et al., 1986) und dabei ein konservativer Aminosäure-Austausch von Valin nach Alanin in Kauf genommen. Diese Mutation wurde sowohl in P1-Cre eingeführt (Fig. 3C), als auch in hCre (Fig. 3D); in hCreV336A sind somit sämtliche kryptischen SD-Stellen am 3'-Ende eliminiert (Fig.3D), in P1-CreV336A ist noch eine SD vorhanden (Fig. 3C).

Unter Verwendung einer loxP-Stop-loxP-lacZ-enthaltenden Reporter-Zell-Linie (Kellendonk et al. 1999) wurden die Konstrukte quantitativ auf ihre Induzierbarkeit, insbesondere Cre-Aktivität in Abwesenheit des Induktors, hin untersucht (Fig. 4). Das durch die V336A-Mutation optimierte hCre-PR-kodierende Konstrukt zeigt tatsächlich einen fast nicht mehr meßbaren Hintergrund (Fig. 4C)- um ca. den Faktor 10 niedriger als das nicht mutierte Konstrukt (Fig. 4B). Dementsprechend verbessert sich die Induzierbarkeit von ca. 10- bis 15-fach auf 76- bis 165-fach. Die Einführung der Mutation V336A in die P1-Cre-Sequenz resultiert in einer Hintergrundaktivität (Fig. 5B), die zwischen der von dem mutierten hCre-Konstrukt (Fig. 5C) und der vom unmutierten P1-Cre (Fig. 5A) liegt. Dies könnte auf die in diesem auf P1-Cre basierenden Konstrukt enthaltenen weiteren kryptischen Spleißstellen zurückzuführen sein (vgl. Fig. 6C). Die maximale Erniedrigung des Hintergrunds rührt von dem Konstrukt mit den wenigsten kryptischen Donorstellen her, nämlich hCreV336A-PR (vgl. Fig. 6D).

Eine weitere Verbesserung konnte in Bezug auf die maximale Aktivität erreicht werden. Die in Fig.4 dargestellten Cre-PR-Fusionsproteine weisen eine maximale Aktivität von lediglich 30-40 % auf - gemessen an konstitutiv aktivem, unfusioniertem Cre. Aus früheren Untersuchungen in unserem Labor war bekannt, daß andere Varianten von Cre-PR (z.B. Cre-PR650-914) eine deutlich höhere Aktivität aufweisen - bis über 50 % von Cre. Allerdings weisen diese Konstrukte immer auch einen sehr hohen Hintergrund auf (siehe Fig. 5D) und sind deswegen für den Einsatz im transgenen Tier kaum geeignet. Durch Einführung der oben beschrieben Mutation V336A in diese Cre-PR-Variante kann der unerwünschte Hintergrund reduziert werden auf wiederum nahezu unmeßbares Niveau bei gleichbleibend hoher Aktivität von über 40 % (Fig. 5E).

Bei der erfindungsgemäßen DNA-Sequenz (wie vorstehend unter (1) definiert) ist die Cre-Proteinsequenz ändernde Basenmutation, durch die die kryptische Spleißstelle in der Cre-DNA-Sequenz ausgeschaltet wird, vorzugsweise ein Basenaustausch, in dem das Kodon GTG in der vorstehend unter (1) definierten kryptischen Spleißstellensequenz durch ein Kodon XYZ ersetzt ist, wobei X, Y und Z unabhängig voneinander die Nukleotide A, T, C oder G sind, mit der Maßgabe, daß wenn X = G, dann Y ≠ T (d. h., wenn Y = T, dann X ≠ G). Dies bedeutet, daß jede Sequenz, die die nicht für den Spleißvorgang kritische Sequenz GT an dieser Position enthält, geeignet ist. Dabei sind insbesondere solche Basensequenzen bevorzugt, die zu einem konservativen Aminosäureaustausch in der resultierenden Proteinsequenz führen. Da die Sequenz GTG für Val kodiert, bedeutet dies, daß insbesondere aliphatische Aminosäuren wie Alanin (XYZ ist GCN), Leucin (XYZ ist CTN, TTA, TTG) und Ile (XYZ ist ATA, ATC oder ATT) kodierende Sequenzen bevorzugt sind. Bevorzugte DNA-Sequenzen weisen die in SEQ ID NOs: 13 und 14 gezeigten Teilsequenzen auf.

Darüber hinaus können in der erfindungsgemäßen DNA-Sequenz noch andere in der Wildtypsequenz vorhandene kryptische Spleißstellen durch stille Mutation und/oder durch die Cre-Proteinsequenz ändernde Basenmutationen ausgeschaltet sein. Geeignete Positionen für solche Mutationen sind Fig. 1 und 6 zu entnehmen.

In einer bevorzugten Ausführungsform der vorliegenden Erfindung ist die für die Cre-Mutante kodierende DNA-Sequenz noch am 5'-Ende, bezogen auf die Cre-Wildtypsequenz, trunkiert. Dabei ist jede Trunkierung, die die Funktionalität des resultierenden Cre-Proteins nicht beeinflußt, geeignet. Es ist jedoch bevorzugt, daß die Nukleotide, die der Position 1 bis 54, vorzugsweise 1 bis 15 des Wildtyps entsprechen, trunkiert sind.

In einer bevorzugten Ausführungsform der vorliegenden Erfindung ist die für die Cre-Mutante kodierende DNA-Sequenz noch am 5'-Ende modifiziert. "Modifiziert" im Sinne der vorliegenden Erfindung bedeutet dabei, daß sowohl zusätzliche Nukleobasen an das 5'-Ende des untrunkierten wie auch an das 5'-Ende des wie vorstehend definierten trunkierten Cre DNA-Sequenz angefügt sein können. Bei diesen zusätzlichen Nukleobasen handelt es sich um Kodons, die für Aminosäuren kodieren, die sich gemäß der sogenannten N-End-Regel stabilisierend auf das Gesamtprotein auswirken sollen (Lévy, F. et al., Proc. Natl. Acad. Sci. USA 93, 4907 - 4912 (1996)). Solche stabilisierende Aminosäuresequenzen bestehen im wesentlichen aus neutralen Aminosäuren, vorzugsweise aus Met, Gly, Val und Ala. Es sind dabei Sequenzen, die Kombinationen dieser Aminosäuren enthalten und homogene Sequenzen einer der genannten Aminosäuren möglich. Die Länge der stabilisierenden Aminosäuresequenzen beträgt vorzugsweise 1 bis 10 Aminosäuren (entspricht 3 bis 30 Nukleobasen), insbesondere 1 bis 3 Aminosäuren. In einer besonders bevorzugten Ausführungsform enthält die DNA-Sequenz die unmittelbar am Translationsstart beginnende Sequenz ATG GGC GCC (kodierend für Met-Gly-Ala).

In einer bevorzugten Ausführungsform umfaßt die erfindungsgemäße DNA-Sequenz die Nukleobasen 1 bis 984 der SEQ ID NO: 5, 7 oder 9.

In einer weiteren bevorzugten Ausführungsform ist die DNA-Sequenz am 3'-Terminus, bezogen auf die Wildtypsequenz, trunkiert. Im Sinne der vorliegenden Erfindung können dabei 1 bis 24 Nukleotide, bezogen auf die in SEQ ID NO: 1 gezeigte Wildtypsequenz, trunkiert sein (d. h. eine Trunkierung nach der in Position 1006 - 1008 befindlichen, die Cre-Proteinsequenz ändernden Mutation). Auch Deletionen einzelner Kodons stromab des Kodons 1006 - 1008 sind gemäß der vorliegenden Erfindung möglich.

In der für die ein Fusionsprotein kodierenden DNA-Sequenz ist das funktionelle Protein ausgewählt aus der Gruppe der Proteine, bestehend aus Enzymen, Peptidhormonen, pharmakologisch wirksamen Peptiden und Strukturproteinen. Die DNA-Sequenzen, die für die funktionellen Proteine kodieren, können dabei sowohl stromaufwärts oder stromabwärts der für die Cre-Mutante kodierenden DNA-Sequenz liegen. Die stabilisierende Wirkung der erfindungsgemäßen Cre-Mutantensequenz kommt jedoch besonders dann zum Tragen, wenn sich die für das funktionelle Protein kodierende DNA-Sequenz stromabwärts der Cre-DNA-Sequenz befindet, d. h. mit dem 3'-Ende der Cre-DNA-Sequenz verknüpft ist.

Vorzugsweise ist das funktionelle Protein die ligandenbindende Domäne eines Rezeptorproteins, insbesondere eine ligandenbindende Domäne eines Stereoidrezeptors wie Progesteron-, Estrogen- oder Glukokortikoidrezeptor. Diese DNA-Sequenzen können dabei von allen Säugern, vorzugsweise von Mensch, Maus oder Ratte stammen. Besonders bevorzugt ist eine ligandenbindende Domäne eines Steroidrezeptors, insbesondere eine, die die Nukleotide 1948 bis 2742 der in SEQ ID NO: 3 gezeigten humanen Sequenz umfaßt.

Die vorstehend definierten bevorzugten für Fusionsproteine kodierenden DNA-Sequenzen können noch weitere Fusionspartner (für funktionelle Proteine kodierende DNA-Sequenzen) enthalten, so z. B. solche, die eine erleichterte Aufreinigung (wie z. B. Glutathion-S-transferase, GST oder maltosebindendes Protein, MBP), Detektion (z. B. grün fluoreszierendes Protein, GFP) oder Transduktion (z. B. Transduktionsdomänen von HIV-TAT oder VP22) ermöglichen. Daneben betrifft die vorliegende Erfindung auch DNA-Sequenzen, die für Fusionsproteine kodieren, die die erfindungsgemäße Cre-Sequenz und die genannten weiteren Fusionspartner umfassen.

Weiterhin ist bevorzugt, daß in dem Fusionsprotein die Linkersequenz zwischen der Cre-Domäne und der für das funktionelle Protein, insbesondere für die ligandenbindenden Domäne kodierende DNA-Sequenz möglichst kurz und/oder mutiert ist, damit die Zugänglichkeit für Proteasen erschwert ist. Für die diese Fusionsproteine kodierenden DNA-Sequenzen bedeutet dies, daß - falls sich die für das funktionelle Protein (bzw. die ligandenbindende Domäne) kodierende DNA stromabwärts der Cre-DNA-Sequenz befindet - der Bereich nach der mutierten kryptischen Spleißstelle (bei Position 1006 - 1008, bezogen auf die Wildtypsequenz) bis zum Ende der Cre-Sequenz bzw. bis zum funktionellen Beginn der ligandenbindenden Domäne, wie vorstehend definiert, ganz oder teilweise deletiert und/oder mutiert sein kann. Unter "mutiert" ist dabei auch zu verstehen, daß die ursprüngliche DNA-Sequenz durch eine kurze DNA-Sequenz ersetzt wird, die für eine Aminosäuresequenz kodiert, die im wesentlichen aus neutralen Aminosäuren wie Gly, Ala, Val, Pro oder Ile, insbesondere Gly und Pro, kodiert. Die Länge der Aminosäuresequenz liegt dabei insbesondere im Bereich von 1 bis 10, vorzugsweise 1 bis 5 Aminosäuren.

Vorzugsweise sind ebenfalls die in der ligandenbindenden Domäne kodierenden Sequenz vorkommenden Spleißstellen durch stille Mutation oder durch eine die Proteinsequenz der ligandenbindenden Domäne ändernden Basenmutation ausgeschaltet. Besonders bevorzugte Sequenzen sind die in den SEQ ID NOs: 5, 7 und 9 gezeigten DNA-Sequenzen.

Der erfindungsgemäße Vektor, wie vorstehend unter (3) definiert, kann neben der DNA-Sequenz (1) und/oder (2) noch weitere funktionelle Sequenzen wie die vorstehend definierten Sequenzen, die die Aufreinigung, Detektion oder Transduktion ermöglichen, enthalten. Weiterhin kann er auch Promotorsequenzen enthalten, die eine Expression z.B. in Bakterien oder Hefen, bzw. Schmetterlingszellen ermöglichen oder eine Expression in transgenen Lebewesen wie vorstehend definiert. Daneben kann der Vektor Replikationsursprünge, Selektionsmarker sowie multiple Klonierungsstellen o. ä. beinhalten.

Die erfindungsgemäßen Mikroorganismen oder transgene Lebewesen können sowohl mit dem vorstehend definierten erfindungsgemäßen Vektor transformiert sein, als auch die erfindungsgemäße DNA-Sequenz chromosomal integriert aufweisen. Als Mikroorganismen im Sinne der vorliegenden Erfindung sind dabei sowohl Prokarionten als auch Eukarionten zu verstehen. "Lebewesen" im Sinne der vorliegenden Erfindung sind dabei Wirbeltiere, insbesondere Säuger (und hiervon insbesondere Nagetiere wie Mäuse oder Ratten) oder Fische oder Nichtwirbeltiere wie Würmer und Fliegen.

Die erfindungsgemäße Cre-Mutante oder das erfindungsgemäße Cre-Fusionsprotein kann neben den vorstehend definierten Sequenzen auch noch weitere proteinogene Strukturen enthalten oder auch mit nichtproteinogenen Strukturen verknüpft sein.

Das erfindungsgemäße Verfahren zur Herstellung der Cre-Mutante oder des Cre-Fusionsproteins kann, wie vorstehend definiert, durch Kultivieren eines mit einem geeigneten Vektor transformierten Mikroorganismusses bewirkt werden. Die Zielproteine können dann gemäß dem Fachmann geläufigen Verfahren aus der Kultur isoliert und aufgereinigt werden.

Die Erfindung hat besondere Bedeutung für die Weiterentwicklung des induzierbaren, konditionalen 'gene-targeting'. Unter konditionalem gene-targeting versteht man die gezielte Mutagenese eines Gens in einem frei wählbaren Zelltyp bzw. Gewebe; die Mutagenese kann ggf. unter Verwendung eines induzierbaren Systems auch zu einem beliebigen Zeitpunkt erfolgen. Diese Modifikationen des klassischen gene-targeting sind insbesondere wichtig, wenn die bereits in Keimzellen auftretende Mutation unerwünschte Nebenwirkungen zeigt. Unter Anwendung der Erfindung können transgene Tiere - vorzugsweise Mäuse - hergestellt werden, die ein posttranslational induzierbares Cre-Fusionsprotein exprimieren. Nach Kreuzung mit einer Mauslinie, welche eine loxP-enthaltende target-Sequenz trägt, können über Zugabe des Induktors zu einem gewünschten Zeitpunkt gezielte Veränderungen im Genom - wie z.B. Deletionen, Inversionen oder Translokationen - vorgenommen werden. Der besondere Vorteil der Erfindung liegt dabei in der beschriebenen niedrigen Cre-Rekombinase-Aktivität vor Zugabe des Induktors. Dadurch ist im transgenen Tier keine unerwünschte (d.h. vor Induktorzugabe auftretende) Modifikation des Genotyps zu erwarten. Eine derartige Cre-Hintergrund-Aktivität erschwert bislang die eindeutige Festlegung des Genotyps vor bzw. nach der Induktion. Je nach Spezifität des im Transgen verwendeten Promotors kann die Expression des induzierbaren Cre entweder zelltyp- bzw. Gewebe-spezifisch oder ubiquitär erfolgen. Im letzteren Fall erhält man ein transgenes Tier, welches die zeitliche Kontrolle über die Mutagenese in jedem Gewebe- bzw. Zelltyp erlaubt. Kontrolle hinsichtlich der Zeit und des Ortes werden durch die Verwendung zelltyp-spezifischer Promotoren ermöglicht. Die Transgenkonstrukte können entweder über Mikroinjektion zur Generierung transgener Linien benutzt werden oder über homologe Rekombination in ausgewählte Bereiche des Genoms integriert werden.

Die vorliegende Erfindung wird anhand der nachfolgenden Beispiele näher erläutert.

### Beispiele

In den nachfolgend beschriebenen Beispielen wurden die folgenden Primer und Matrizen verwendet:

Primer:
3Bsicre343 | AAATTCGTACGCATCGCCATCTTCCAGCAGG
3Narcre343 | AAATTGGCGCCATCGCCATCTTCCAGCAGG
3Kascre343 | AATTTGGCGCCGTCCCCATCCTCGAGCAG
3Sfomutcre343 | AAATTGGCGCCATCGCCATCTTCCAGCAGGCGCGCCATT

GCCCC
3Sfomutsshcre343 | AAATTGGCGCCGTCCCCATCCTCGAGCAGCCTCGCCATG
GCCCC
5Bamhcre2 | GGGGGATCCACCATGGGTGCCTCCAACCTGCTGACTGTG
5Bamhcre19 | TTTAAGGATCCACCATGGGTGCCACGAGTGATGAGGTTC
GCA
5Bsicre19 | TTTAACGTACGGCACGAGTGATGAGGTTCGCA
5Narcre19 | TTTAAGGCGCCACGAGTGATGAGGTTCGCA

| Matrizen: | |
|---|---|
| pGKCrebpA | Institut für Genetik, Universität zu Köln. |
| pBluehCre | Seeburg, MPI für med. Forschung, |
| | Heidelberg & Stewart, EMBL, Heidelberg. |
| pNNCre19PR676-914 | Institut für Genetik, Universität zu Köln. |
| pNNhCre19PR676-914 | Institut für Genetik, Universität zu Köln. |
| pNN265E-bpA | Artemis, Köln. |

### Beispiel 1: Klonierungsschritt I: LBD in pNN

Im ersten Klonierungsschritt wurden die verschiedenen Längen der LBD des PR und des ER in pNN265E⁻bpA (erhältlich Institut für Genetik, Univ. zu Köln) kloniert. Die Klonierung wurde über die im Vektor nur einmal schneidenden Restriktionsenzyme BamHI und EcoRV durchgeführt, die über PCR auch in die Amplifikate eingeführt wurden.

Unterschiedliche Längen der LBD des PR wurden durch 5'- und 3'-terminale Trunkierungen generiert. Die 5'-Trunkierungen begannen bei AS650, AS 676 und AS678 des PR. Durch Beginn der PR-LBD bei Leu650 wird die D-Domäne der LBD um 10 AS verkürzt. Dieser N-Terminus wurde gewählt, da solche Konstrukte in Kontext mit Cre erfahrungsgemäß hohe Aktivitäten aufweisen (Kellendonk et al., Nucl. Acid Res. 24, 1404-1411 (1996)). Eine zweite N-terminale Trunkierung erfolgte bis zu Ser676 des PR. Dies basierte auf dem Befund von Kellendonk et al. (1996) und daraus resultierende Überlegungen: Je weniger AS der D-Domäne des PR als Linker zwischen Cre und LBD, desto geringere Hintergrundaktivität der Fusionsproteine. Es wurden u. a. die AS 641, 672 und 687 als Startpunkte für die LBD getestet (Kellendonk et al., 1996). PR641 zeigte in Kontext mit Cre hohe Aktivität, aber auch hohen Hintergrund. Dagegen zeigte PR672 etwas weniger Aktivität und auch weniger Hintergrund als PR641. PR687 zeigte kaum noch Aktivität. Daher wurde der Startpunkt des Ser676-Konstrukts zwischen AS672 und AS687 gelegt, in der Erwartung, daß eine hohe Aktivität von Cre beibehalten wird und auch der Hintergrund auf tolerierbarem, niedrigem Niveau bleibt. Zusätzlich sind bei Ser676-Konstrukten im Gegensatz zu Leu650 auf Sequenzebene zwei starke Spleiß-Akzeptoren (SA) für mögliches kryptisches Spleißen abgewandelt worden. Ein SA wird durch Deletion der Sequenz eliminiert. Der zweite SA wird derart in seiner Konsensus-Sequenz verändert, daß der C/T-reiche Sequenzabschnitt in diesem Konstrukt fehlt. Zudem wurde im Linkerbereich darauf geachtet, mögliche Proteaseerkennungs-AS wie z. B. Arg, Lys zu deletieren. Bei der dritten 5'-Trunkierungsvariante Gly678 wurde die restliche Sequenz des zweiten SA, die in Ser 676 noch vorhanden ist, deletiert. Um Cre-Aktivität in solchen Konstrukten durch Verkürzung des Linkers nicht noch weiter einzuschränken, wurden anstatt des Ser676 und des Pro677 zwei Glycine eingeführt, die auch die Flexibilität des Linkers steigern sollen.

### Beispiel 2: Klonierung von Cre19 an den N- oder C-Terminus der LBD

Die ungerichtete Klonierung von Cre19 an den N-Terminus der LBD erfolgte über die KasI-Schnittstelle. Das an der Matrize pGKCrebpA amplifizierte Insert (Tab. 1) wurde mit KasI verdaut und in KasI geschnittene und danach dephosphorylierte pNN-Vektoren mit LBD einligiert. Zudem wurde Cre19 an das C-terminale Ende der LBD ungerichtet über die BsiWI-Schnittstelle der pNN-Vektoren mit den verschiedenen LBD des PR und ER kloniert. Hierzu wurde die Cre19-Sequenz über PCR mit den in Tab. 1 dargestellten Primern an der Matrize pGKCrebpA amplifiziert, mit BsiWI geschnitten und in BsiWI geschnittene, dephosphorylierte pNN-Vektoren einligiert.

**Tabelle 1:**

| verwendete Primersequenzen | | | |
|---|---|---|---|
| | 5'-Primer | 3'-Primer | Matrize |
| N-terminales Cre19 | 5Narcre19 | 3Narcre343 | pGKCrebpA |
| c-terminales Cre19 | 5Bsicre19 | 3Bsicre343 | pGKCrebpA |

### Beispiel 3: Klonierung des hCre an den N-Terminus von PR676-914

Da Cre ein Phagenprotein ist und die Sequenz dadurch auf die prokaryotische Translationsmaschinerie abgestimmt ist, wurde versucht, eine Optimierung für das eukaryontische Expressionssystem zu erzielen. R. Sprengel optimierte in Zusammenarbeit mit F. Stewart die Sequenz von Cre entsprechend, um somit eine verbesserte Translationseffizienz zu erhalten. Die so veränderte Cre-Sequenz nannte er humanisiertes Cre (hCre) (F. Stewart, unveröffentlichte Daten). Nachdem sich herausstellte, daß das Konstrukt pNNCre19PR676-914 in Zellkultur die bisher besten Cre-Aktivitäten zeigte, wurde in diesem Konstrukt an Stelle des normalen Cre19 ein hCre eingefügt.

Die Sequenzen von hCre2 und hCre19 wurden jeweils N-terminal an PR676-914 gerichtet kloniert. Die PCR des hCre19 und hCre2 wurde mit den in Tab. 2 angegebenen Primern an der Matrize pBluehCre vorgenommen, die Amplifikate BamHI/KasI geschnitten und in einen BamHI/KasI geschnittenen pNNCre19PR676-914 ligiert.

**Tabelle 2:**

| verwendete Primersequenzen | | | |
|---|---|---|---|
| | 5'-Primer | 3'-Primer | Matrize |
| N-terminales hCre2 | 5Bamhcre2 | 3Kascre343 | pBluehCre |
| N-terminales hCre19 | 5Bamhcre19 | 3Kascre343 | pBluehCre |

### Beispiel 4: Klonierung des Cre19V336A

Die Mutation V336A wurde in die Phagen-Cre19-Sequenz eingeführt. Dazu wurde die Cre-Sequenz mit dieser Mutation, nachfolgend Cre19V336A genannt, N-terminal an die PR676-914-Sequenz kloniert.

Die Mutation V336A wurde durch den in Tab. 3 aufgeführten 3'-Primer in die Cre19-Sequenz eingeführt. Das Amplifikat wurde an der Matrize pNN-Cre19PR676-914 amplifiziert und über die SfoI-Schnittstellen in einen SfoI geschnittenen, nachfolgend dephosphorylierten pNNCre19PR676-914 ungerichtet kloniert. Durch Verdau mit BamHI konnte die richtige Orientierung des Cre19V336A im Vektor gezeigt werden. Die Mutation wurde über Sequenzierung nachgewiesen.

**Tabelle 3:**

| verwendete Primersequenzen | | | |
|---|---|---|---|
| | 5'-Primer | 3'-Primer | Matrize |
| Cre19V336A | 5Narcre19 | 3Sfomutcre343 | pNNCre19PR676-914 |

### Beispiel 5: Klonierung des hCre19V336A

Die Mutation V336A wurde über den in Tab. 4 aufgeführten 3'-Primer in die Sequenz des hCre19 eingeführt. Über die mittels PCR an der Matrize pNNhCre19PR676-914 eingeführten Klonierungsstellen BamHI und SfoI wurde das hCre19V336A in einen BamHI/SfoI geschnittenen pNNCre19PR676-914 kloniert. Die Mutation wurde über Sequenzierung nachgewiesen.

**Tabelle 4:**

| | 5'-Primer | 3'-Primer | Matrize |
|---|---|---|---|
| hCre19V336A | 5Bamhhcre19 | 3Sfomutsshcre343 | pNNhCre19PR676-914 |

### Beispiel 6: Aktivitätstest von Cre19-PR676-914, hCre19-PR676-914 und hCre19V336A-PR676-914

Es wurden am Vortag in Löchern von 6-Loch-Platten (8X10⁴Zellen/6-Loch/Transfektion) ausgesäte CV1-5B-Zellen im Triplikat mit 1 µg der jeweiligen Fusionsproteinplasmide und 1 µg pHD2-AP transfiziert. 12 h nach Transfektion wurden die Zellen abtrypsiniert und zu gleichen Teilen auf 2 Löcher einer 12-Loch Platte aufgeteilt. Das eine Loch wurde mit 100 nM RU486 (erhältlich von Sigma Chemie GmbH, Deisenhofen, Deutschland) in Medium versetzt, das andere mit Leer-Medium. Drei Tage nach Transfektion wurden die Zellen fixiert und gegen β-Galactosidase und Alkalische Phosphatase (zur Normalisierung gegen Transfektionseffizienz) gefärbt. Der prozentuale Abgleich erfolgte gegen parallel transfizierten pNN-CMV-Cre (Cre ohne Fusionspartner im gleichen Vektor). Dazu wurde der Mittelwert von CMV-Cre auf 100% gesetzt und die Mittelwerte für die Fusionsproteine daran abgeglichen. Das Ergebnis ist in Figur 4 zusammengefaßt.

## Patentansprüche

1. DNA-Sequenz, die für eine Mutante der Bakteriophagen-P1-Rekombinase Cre kodiert, in der die kryptische Spleißstelle in der Sequenz ATG GTG CGC, die der Position 1003 - 1011 in der in SEQ ID NO:1 gezeigten Wildtypsequenz entspricht, durch eine die Cre-Proteinsequenz ändernde Basenmutation ausgeschaltet ist.

2. DNA-Sequenz gemäß Anspruch 1, wobei das Kodon GTG in der genannten Spleißstellensequenz, durch ein Kodon XYZ ersetzt ist, wobei X, Y und Z unabhängig voneinander die Nuleotide A, T, C oder G sind, mit der Maßgabe, daß wenn X = G, dann Y ≠ T.

3. DNA-Sequenz gemäß Anspruch 2, wobei das Kodon XYZ für eine neutrale Aminosäure kodiert und insbesondere das Ala kodierende Kodon GCG ist.

4. DNA-Sequenz gemäß einem oder mehreren der Ansprüche 1 bis 3, in der andere in der Wildtypsequenz vorhandene kryptische Spleißstellen durch stille Mutation und/oder durch eine die Cre-Proteinsequenz ändernde Basenmutationen ausgeschaltet sind.

5. DNA-Sequenz gemäß einem der Ansprüche 1 bis 4, die am 5'-Terminus, bezogen auf die Cre-Wildtypsequenz, trunkiert ist, insbesondere eine DNA-Sequenz, in der die Nukleotide, die der Position 1 bis 54 des Wildtyps entsprechen.

6. DNA-Sequenz gemäß einem der Ansprüche 1 bis 5, wobei die DNA-Sequenz am 5'-Ende für zusätzliche, in der Wildtyp-Cre-Sequenz nicht vorhandene Aminosäuren kodierende Nukleotide aufweist.

7. DNA-Sequenz gemäß Anspruch 6, wobei die zusätzlichen Nukleotide für neutrale Aminosäuren, insbesondere für Met, Gly, Val und Ala, kodieren.

8. DNA-Sequenz gemäß einem oder mehreren der Ansprüche 1 bis 7, wobei die DNA-Sequenz am 3'-Terminus, bezogen auf die Wildtypsequenz, trunkiert ist.

9. DNA-Sequenz gemäß Anspruch 1, wobei die DNA-Sequenz die Nukleotide 1 bis 984 der SEQ ID NOs: 5, 7 oder 9 umfaßt.

10. DNA-Sequenz, die für ein Fusionsprotein aus der Bakteriophagen-P1-Rekombinase Cre und einem weiteren funktionellen Protein kodiert, wobei die die Bakteriophagen-P1-Rekombinase Cre kodierende Sequenz wie in Ansprüchen 1 bis 9 definiert ist.

11. DNA-Sequenz gemäß Anspruch 10, wobei das funktionelle Protein eine ligandenbindende Domäne eines Rezeptorproteins ist, insbesondere der Rezeptor ein Stereoid-Rezeptor, bevorzugt ein Progesteron-, Östrogen- oder Glucocorticoid-Rezeptor ist.

12. DNA-Sequenz gemäß Anspruch 11, wobei der Rezeptor ein Progesteronrezeptor ist, insbesondere ein Progesteronrezeptor, der die Nukleotide 1948 bis 2742 der SEQ ID NO: 3 umfaßt.

13. DNA-Sequenz gemäß einem der Ansprüche 10 bis 12, wobei in der für die ligandenbindende Domäne kodierende Sequenz kryptische Spleißstellen durch stille Mutation oder durch eine die Proteinsequenz der ligandenbindenden Domäne ändernde Basenmutation ausgeschaltet sind.

14. DNA-Sequenz gemäß Anspruch 10, die die in SEQ ID NOs:5, 7 oder 9 gezeigte Sequenz aufweist.

15. Vektor, enthaltend eine DNA-Sequenz gemäß einem oder mehreren der Ansprüche 1 bis 14.

16. Mikroorganismus oder transgenes Lebewesen, enthaltend einen Vektor, wie in Anspruch 15 definiert und/oder eine DNA-Sequenz wie in Ansprüchen 1 bis 14 definiert.

17. Cre-Mutante oder Cre-Fusionsprotein, das von der DNA-Sequenz gemäß Ansprüchen 1 bis 14 kodiert wird.

18. Verfahren zur Herstellung einer Cre-Mutante oder eines Cre-Fusionsproteins, wie in Anspruch 17 definiert, umfassend das Kultivieren eines Mikroorganismusses oder einer Zellkultur, der/die mit einem Vektor gemäß Anspruch 15 transformiert oder transfiziert ist.

19. Verwendung einer DNA-Sequenz gemäß Ansprüchen 11 bis 14 zur Mutagenese und/oder Rekombination von loxP-Stellen enthaltende Zielsequenzen in Lebewesen.
